# EUROPEAN PATENT APPLICATION

(11) **EP 3 628 652 A1**
(43) Date of publication of application: **01.04.2020**
(21) Application number: 18197409.8
(22) Date of filing: 28.09.2018
(51) Int. Cl.: C07B 55/00, C07B 59/00

(54) **METHOD FOR THE STEREOISOMERIZATION OF CHIRAL COMPOUNDS**

(71) Applicant: Eberhard Karls Universität Tübingen, 72074 Tübingen (DE)
(72) Inventor: Horak, Jeannie, 72076 Tübingen (DE); Lämmerhofer, Michael, 72070 Tübingen (DE)
(74) Representative: Kuttenkeuler, David

(57) **Abstract**

The present invention provides a novel method for the controlled stereoisomerization (comprising the stereo-conversion of chiral compounds to racemates, non-racemic mixtures and mixtures of epimers). The method of the invention provides the fully controlled generation of mixtures of different ratios of (S) and (R) stereoisomers, both enantiomers and diastereomers, of a given compound or a mixture of compounds. Such mixtures and isotopically labeled variants of said mixtures provided by the present invention are useful as an authenticity or external or internal standards in various biochemical and medical applications. Further, with the method of the invention it is possible to generate specific stereoisomers from some amino acids as well as degradation and oxidation products thereof which were previously not available. Thence, the invention provides in additional aspects the generation of a group of previously unavailable stereoisomers (enantiomers, epimers, and mixtures thereof such as a racemate) with amino acid type structures.

## Description

### FIELD OF THE INVENTION

The present invention provides a novel method for the controlled stereoisomerization (comprising the stereo-conversion of chiral compounds to racemates, non-racemic mixtures and mixtures of epimers). The method of the invention provides the fully controlled generation of mixtures of different ratios of (S) and (R) stereoisomers, both enantiomers and diastereomers, of a given compound or a mixture of compounds. Such mixtures and isotopically labeled variants of said mixtures provided by the present invention are useful as an authenticity or external or internal standards in various biochemical and medical applications. Furthermore, with the method of the invention it is possible to generate specific stereoisomers from some amino acids as well as degradation and oxidation products thereof which were previously not available. Thence, the invention provides in additional aspects the generation of a group of previously unavailable stereoisomers (enantiomers, epimers, and mixtures thereof such as a racemate) with amino acid type structures.

### BACKGROUND

In the continuously expanding field of the so called "omics" analyses', such as proteomics, glycomics, lipidomics or metabolomics, high sample throughput and ultra-fast separation are essential, necessitating reasonably fast and simple sample preparation techniques. Ultra-high performance liquid chromatography coupled with high resolution mass spectrometry (UHPLC-HRMS) combines fast analyte separation with highest possible mass-to-charge (m/z) accuracy. In case of a direct infusion HRMS analysis approach, omitting a (chromatographic) pre-separation of analytes, internal standards preferably isotopically labeled internal standards are essential for accurate quantitative analysis. Concerning the LC-MS analysis of real samples, the dilute and shoot approach resembles the fastest sample preparation technique, since it only includes a simple sample dilution step prior to LC-MS analysis. Hence this approach has gained increasing popularity in LC-MS analysis, ranging from the analysis of drugs and metabolites to large proteinogenic molecules such as insulin and other biopharmaceuticals. The use of isotopically labelled internal standards is of utmost importance to ensure highest possible accuracy in quantitative analytics, because matrix effects are naturally compensated since the isotopically labelled compounds share identical chemical properties with their un-labelled counterparts thus showing identical (eg ¹³C or ¹⁵N labeling) or very similar retention (eg ²H labeling) as the target analytes, and are subject to the same ion-suppression or ion-enhancement in electrospray ionization (ESI) most commonly utilized as interface between LC and MS. An upcoming research field within the omics community is "chiral metabolomics", especially amino acid metabolomics. In the absence of a mass spectrometric detector to determine the mass-to-charge difference between co-eluting sample amino acids and isotopically labelled internal standard, many studies still apply external calibration, or use a non-proteinogenic amino acid as internal standards such as racemic norleucine, since isotopically labelled L-amino acid standards mixtures are costly or not available and chiral isotopically labelled D-amino acid standard mixtures are mostly not available. In order to combine a dilute and shoot approach with chiral separation, the availability and applicability of isotopically labelled amino acid standards for accurate quantitative analysis is essential.

Up to date only partially complete mixtures of ¹³C¹⁵N-L-amino acids are available, omitting the acid labile and decomposition prone amino acids glutamine, asparagine and tryptophan. Since the determination of amino acid composition of peptides and proteins, involve acid or base catalysed hydrolysis to their corresponding free amino acids, glutamine or asparagine deamidate and are therefore absent in the resulting hydrolysates. Hence, the commercial ¹³C¹⁵N-L-amino acid mixture while being applicable for non-chiral amino acids, are only partially applicable for chiral amino acid quantification of protein hydrolysates, but not applicable for the chiral analysis of all free amino acids present in biological samples, such as glutamine and asparagine. Considering that D-glutamic acid is of neurological interest and glutamine is of metabolic interest, especially in cancer research, for cancer cells show a high consumption of glutamine, "accurate" quantification and comparison of free glutamine and glutamic acid is of importance. Hence the availability or straightforward preparation of an isotopically labelled chiral amino acid standard, containing all proteinogenic amino acids including some uncommon amino acids such as allo-threonine, allo-isoleucine, citrulline, theanine or hydroxyproline, just to mention a few is an urgent demand. Concerning their preparation, the most common and widely used approach is the N-terminal racemization of amino acids and amino acid amide via Schiff base formation with aromatic aldehydes in glacial acetic acid and elevated temperatures.

Alternatively also C-terminal racemization of N-acetyl amino acids via oxazolone formation and base induced ring-opening was reported in literature. Furthermore, several patents illustrate the racemization of N-acyl, N-phenacetyl, N-benzoyl - and N-phenacetyl-phosphino amino acid derivatives as well as N-phenacyl-hydroxyproline in the presence of the corresponding acid and heat treatment up to 200°C. The major disadvantage of these methods is, as previously mentioned, the decomposition of several amino acids during acid treatment and heat treatment. In general, epimerization of amino acids within or at the terminal end of a peptide chain depends on the predisposition of an amino acid to epimerize, on their location within the peptide chain as well as on the properties of neighbouring amino acid groups. Concerning N-terminally modified peptides, acetylated Gly-L-Leu was reported to racemize under acidic condition, while their corresponding N-Acetyl-L-Leu-Gly did not racemize, which was proposed as a possible tool to determine the N-terminal amino acids.

For non-derivatized dipeptides Steinberg, S.; et al. Science (Washington, DC, United States 1981, 213, 544-545) observed a C-terminal epimerization as well as sequence inversion due to 1,4-piperazine-2,5-dione (diketopiperazine) intermediate formation. Overall, not much information is available on the chiral separation of dipeptides. However, dipeptides were found in alcoholic beverages, where they are proposed to influence the taste of sake, beer and wine and are used as building blocks for the synthesis of potential anti-tumor agents. While the D-amino acid content of biological fluids may be very low (<1%), certain non-ribosomal peptides on the other hand can contain predominantly D-amino acids. Similarly, therapeutic peptides may contain D-amino acids as a strategy to increase metabolic stability. Hence it would be beneficial to be able to prepare isotopically labelled chiral amino acid mixtures with tailored amount of isotopically labelled amino acids ranging from 1-5% D-amino acids up to 25% to 50%, depending on the amino acid type and analytical application.

Therefore, it was an object of the present invention to provide a novel method for the generation of stereoisomers such as enantiomers and mixtures thereof of a predetermined ratio, such as a racemic, non-racemic or stereoisomer mixture. It was one additional object of the invention to provide enantiomers of compounds that were, although theoretically possible, up to date not obtainable by any means of the prior art, nor naturally occurring.

### BRIEF DESCRIPTION OF THE DRAWINGS

The invention will be better understood with reference to the detailed description when considered in conjunction with the non-limiting examples and the accompanying drawings, in which:

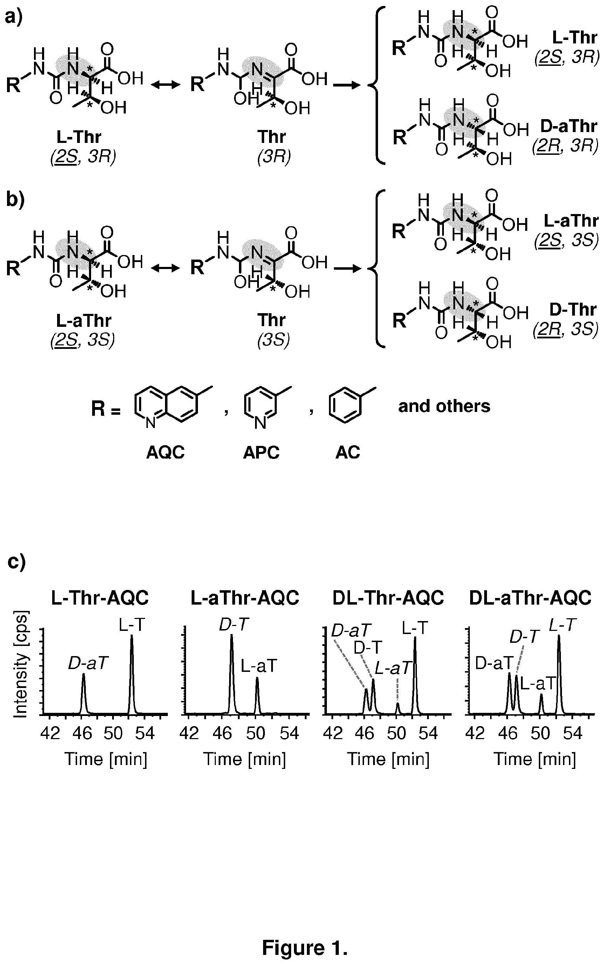

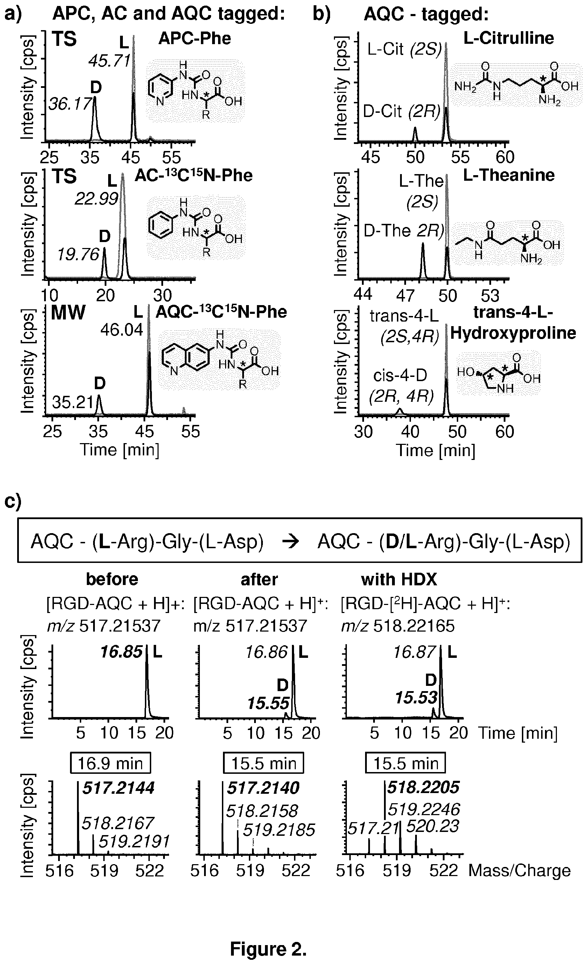

**Fig. 1** shows the proposed stereoisomerisation mechanism of amino acids tagged with a stereoisomerisation tag **R** shown for threonine, depicted for **Fig. 1a** L-Thr and **Fig.1b** L-aThr. Stereoisomerisation only occures in position 2. The second stereogenic center in position 3 for Thr and aThr is not affected. Examples for stereoisomerisation tags R are shown, namely 6-aminoquinolyl-N-hydrosysuccinimidyl carbamate (AQC), 3-aminopyridyl N-hydroxysuccinimidyl carbamate (APC) and aminophenyl-N-hydroxysuccinimidyl carbamate (AC). Application examples for APC and AC-tags are shown in **Fig.2a****.** **Fig. 1c** shows the chromatographic results for the stereoisomerisation of Thr-AQC and aThr-AQC. Enantiomer separation was performed on a Chiralpak ZWIX(+) column (150 x 4 mm, 3 µm) using the long hydro-organic gradent elution method with TOF-ESI-MS detection in positive mode. Extracted ion chromatograms (XICs) of isobaric compounds Thr and aThr ([Thr-AQC + H]+: *m*/*z* 290.11408) after stereoisomerisation of L-Thr, L-aThr, DL-Thr and DL-aThr, which provide the stereoisomeric mixtures D-aThr and L-Thr; D-Thr and L-aThr; D-aThr, D-Thr, L-aThr and L-Thr; and D-aThr, D-Thr, L-aThr and L-Thr, respectively. Experimental conditions are shown in **Example 1.**
**Fig. 2a** shows the extracted ion chromatograms (XICs) are for the three stereoisomerisation tags 3-aminopyridyl N-hydroxysuccinimidyl carbamate (APC), aminophenyl-N-hydroxysuccinimidyl carbamate (AC) and 6-aminoquinolyl-N-hydroxysuccinimidyl carbamate solution (AQC). For the APC-tag and AC-tag, stereoisomerisation was performed with a thermoshaker (TS), while for the AQC-tag an example for stereoisomerization using microwave irradiation (MW) is shown. APC-Phe ([M+H]⁺: *m*/*z* 286.1186), AC-*u*-¹³C¹⁵N-Phe ([M+H]⁺: *m*/*z* 295.1506), and AQC-*u*-¹³C¹⁵N-Phe ([M+H]⁺: *m*/*z* 346.1615). Note that the XICs of the original L-stereoisomers are shown in gray and XICs of the stereoisomersised samples are shown in black. Enantiomer separation was performed on a Chiralpak ZWIX(+) (150 x 4 mm, 4 µm) column using the long hydro-organic dual gradient method with UHPLC-QTOF-ESI-MS in positive ionisation mode. Sample preparation provided in **Example 2.**
**Fig. 2b** shows the extracted ion chromatograms (XICs) for AQC-tagged L-citrulline ([Cit-AQC+H]⁺: *m*/*z* 346.1510), L-theanine ([The-AQC+H]⁺: *m*/*z* 345.15570) and trans-4-L-hydroxy-proline ([Hyp-AQC+H]⁺: *m*/*z* 302.1135) after stereoisomerisation. Note that trans-4-L-Hyp (2S, 4R) provided cis-4-D-Hyp (2R, 4R) and cis-4-L-Hyp (2S, 4S) provided trans-4-D-Hyp (2R, 4S) (data not shown).). Also note that the XICs of the original L-stereoisomers are shown in gray and XICs of the stereoisomersised samples are shown in black. Enantiomer separation was performed on Chiralpak ZWIX(+) column (150 x 4 mm, 3 µm) using the long hydro-organic dual gradient method with UHPLC-QTOF-ESI-MS in positive ionisation mode. Sample preparation provided in **Example 3.**
**Fig. 2c** shows the extracted ion chromatograms (XICs) of [AQC-(D/L-Arg)-Gly-(L-Asp) + H]⁺: *m*/*z* 517.21537 and [AQC-(D/L-[²H]-Arg)-Gly-(L-Asp) + H]⁺: *m*/*z* 518.22165 after stereoisomerization. Enantiomer separation of AQC-(L-Arg)-Gly-(L-Asp) and AQC-(D-Arg)-Gly-(L-Asp) was performed with the short gradient elution method on the Chiralpak ZWIX (+) (150 x 4 mm, 3 µm) column with an injection volume of 1 µL. Sample preparation provided in **Example 4.**

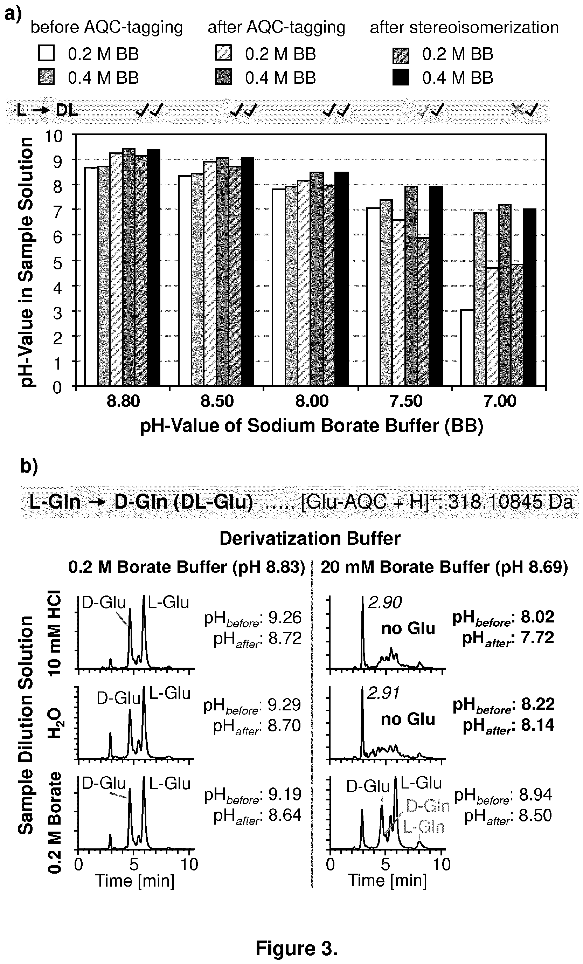

**Fig. 3a** shows an overview of in-solution pH values measured before AQC-tagging, after AQC-tagging and after stereoisomerisation of a *u*-12C, 14N-L-amino acid (mixture of all 20 amino acids) solution employing 0.2 M or 0.4 M sodium borate buffer at pH values of 8.8, 8.5, 8.0, 7.5 and 7.0 for derivatization, followed by purging with nitrogen and stereoisomerisation at 95°C for 15 h using a thermoshaker. Stereoisomerisation was determined with the exemplary extracted ion chromatograms (XICs) of Phe-AQC ([Phe-AQC+H]⁺: *m*/*z* 336.13427). In addition, successful stereoisimerisation was indicated by a hook and failed stereoisomerisation by a cross. Sample preparation provided in **Example 5**
**Fig. 3b** shows the extracted ion chromatograms (XICs) of AQC-tagged glutamic acid ([Glu-AQC + H]⁺: *m*/*z* 318.10845), after stereoisomerisation of glutamine in 20 mM and 0.2 M borate buffer solutions of various pH values, pH 8.69 and pH 8.83 respectively. In addition, the sample solutions were diluted by a factor of 10 with 0.1 M hydrochloric acid, ultra-pure water and 0.2 M borate buffer (pH 8.83). Enantiomer separation was performed on a Chiralpak ZWIX(+) column (150 x 4 mm, 3 µm) with the short gradient elution method using UHPLC-QTOF-ESI-MS in positive ionisation mode. Sample preparation provided in **Example 6.**

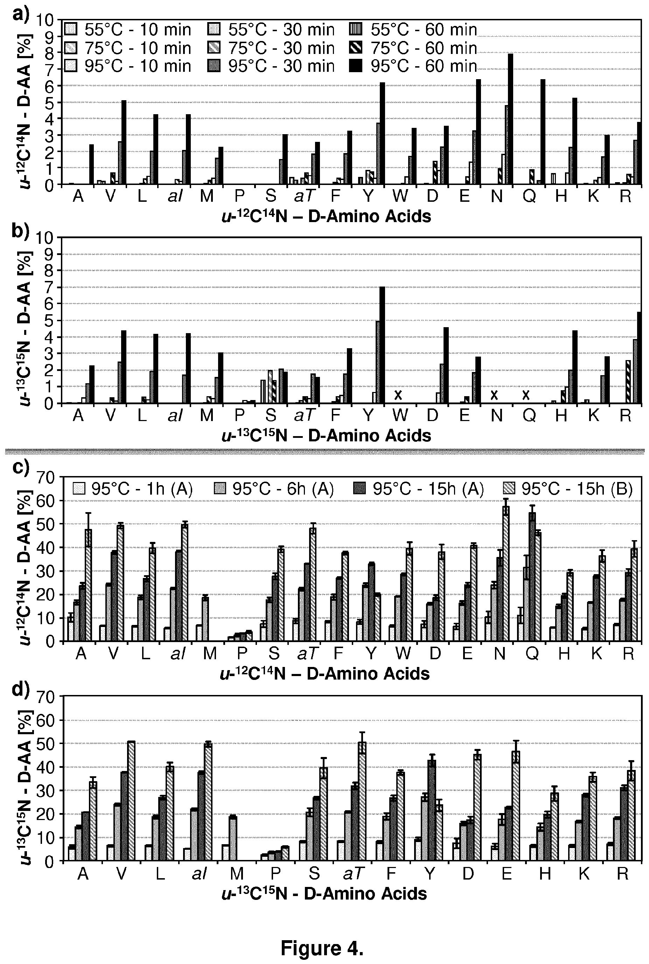

**Fig. 4** shows the stereoisomerisation results of an AQC derivatised mixture of *u*-¹²C¹⁴N amino acids **(****Fig. 4a** and **Fig. 4c****)** and *u*-¹³C¹⁵N amino acids without Asn, Gln and Trp **(****Fig. 4b** and **Fig. 4d**). In **Fig. 4a** and **Fig. 4b** stereoisomerisation (n=1) was performed at 10 min (55°C, 75°C and 95°C), 30 min (55°C, 75°C and 95°C) and 1 h (55°C, 75°C and 95°C), and in **Fig. 4c** and **Fig. 4d** stereoisomerisation (n=3) was performed at 95°C for **1** h, 6 h and 15 h. With condition A samples were analyzed with additional derivatization with AQC after stereoisomerisation, while condition B samples were analyzed without additional derivatization with AQC. The latter showed that the AQC tag was mostly cleaved from the L-enantiomer and not from the stereoisomerised D-enantiomers. Enantiomer separation was performed on Chiralpak ZWIX(+) column (150 x 4 mm, 3µm) using the long hydro-organic dual gradient UHPLC-QTOF-ESI(+)-MS method. Peak integration was performed with the Sciex MultiQuant™ software. Sample preparation provided in **Example 7** and **Example 8.**

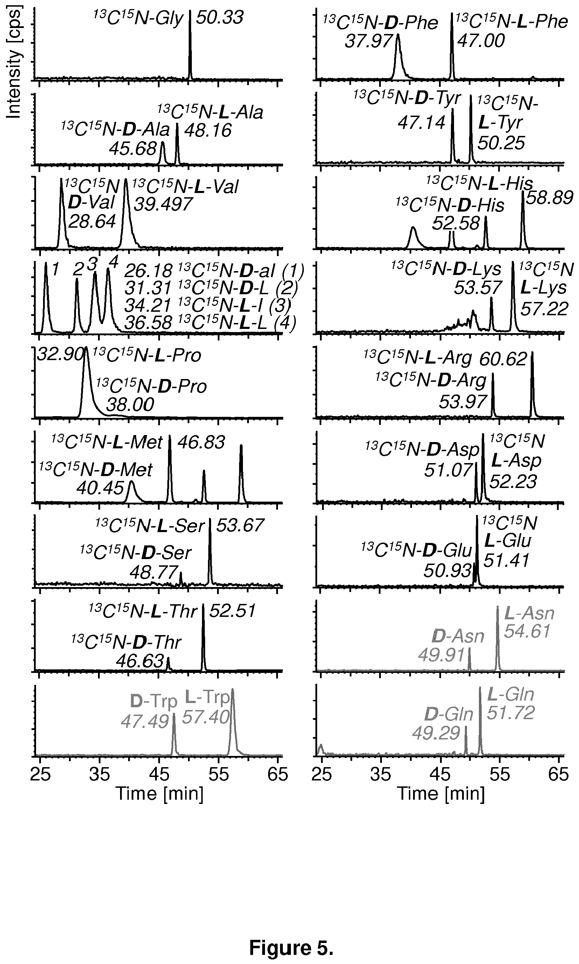

**Fig. 5** shows the extracted ion chromatograms (XIC) of the TOF-MS experiment of the corresponding AQC-tagged *u*-¹³C¹⁵N-L-amino acids (in black), after stereoisomerisation for 6 hours at 95°C. In case of the missing amino acids in the Metabolomics Amino Acid Mix from Euroisotop, Asn, Gln and Trp, the stereoisomerisation results of the corresponding AQC-tagged *u*-¹²C¹⁴N-L-amino acids are shown in grey. Enantiomer separation was performed on a Chiralpak ZWIX(+) column (150 x 4 mm, 3 µm) using the long hydro-organic dual gradient elution method with UHPLC-QTOF-ESI-MS operated in positive ionization mode. Sample preparation provided in **Example 9.**

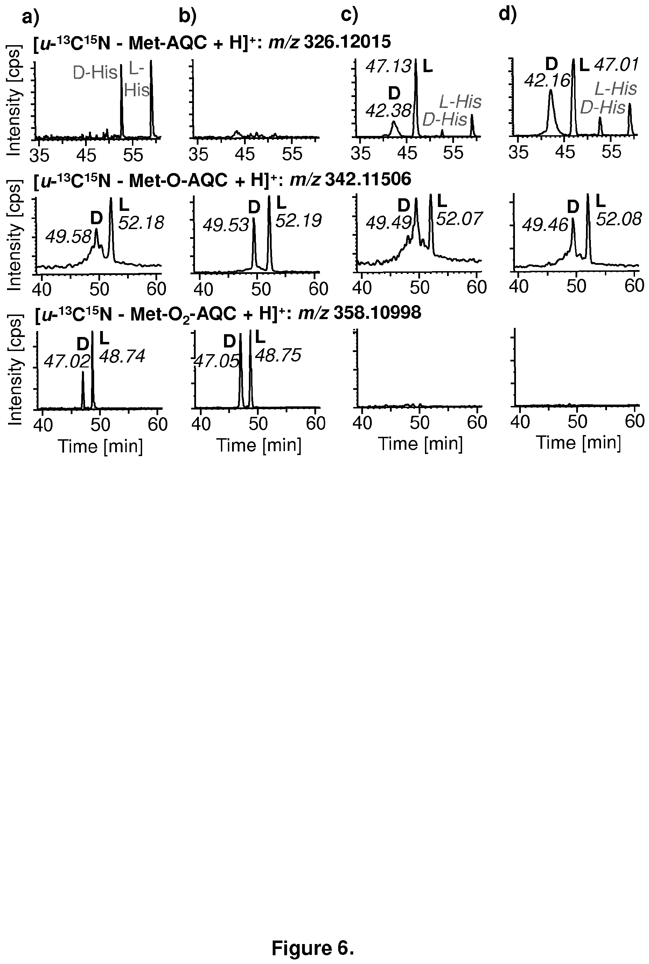

**Fig. 6** shows the extracted ion chromatograms (XICs) of AQC-tagged *u*-¹³C¹⁵N-methionine ([*u*-¹³C¹⁵N-Met-AQC + H]⁺: m/z 326.12015) and its AQC-tagged oxidation products *u*-¹³C¹⁵N-methionine sulfoxide-AQC ([u-¹³C¹⁵N-Met-(O)-AQC + H]⁺: m/z 342.11506) and u-¹³C¹⁵N-methionine sulfone-AQC ([*u*-¹³C¹⁵N-Met-(O)₂-AQC + H]⁺: m/z 358.10998) after stereoisomerisation at 95°C for a) 6 h and b) 24 h (0.25 mM *u*-¹³C¹⁵N amino acid mixture) in the presence of oxygen; and for c) 3 h and d) 12 h after ultrasonication and purging with nitrogen. Note that *u*-¹²C¹⁴N-histidine-AQC ([*u*-¹²C¹⁴N-His-AQC + H]⁺: m/z 326.12531) is isobaric to u-¹³C¹⁵N-methionine-AQC. Enantiomer separation was performed on Chiralpak ZWIX(+) column (150 x 4 mm, 3µm) using the long hydro-organic dual-gradient elution UHPLC-QTOF-ESI-MS method in positive ionisation mode. Elution order was verified for Met, but was assumed for Met(O)-AQC and Met(O)₂-AQC, since on Chiralpak ZWIX(+), D-enantiomers tend to elute before L-enantiomers, except for Pro-AQC. Sample preparation provided in **Example 10.**

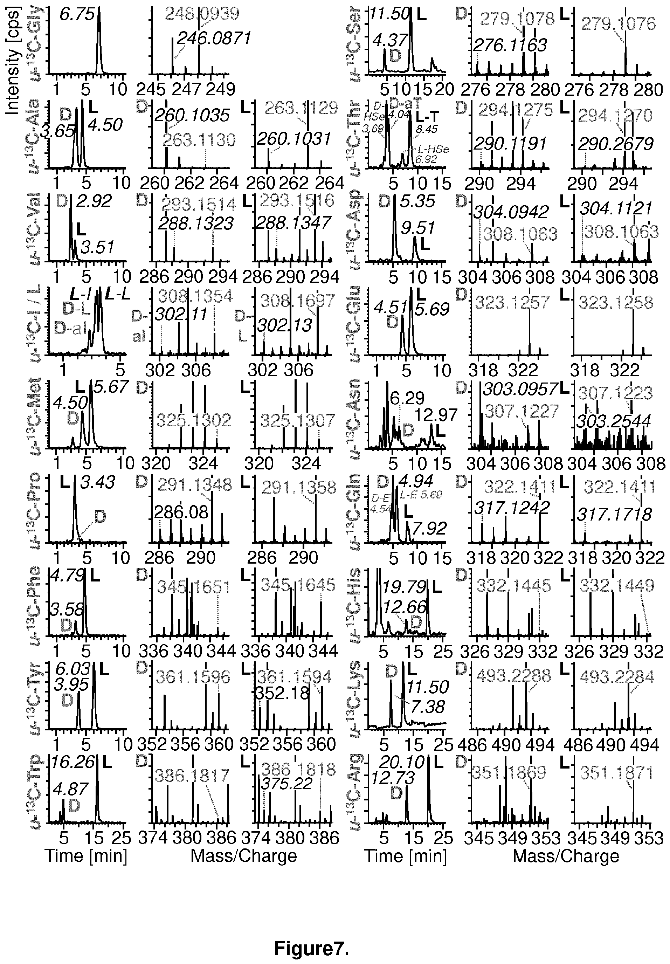

**Fig. 7** shows the enantiomer separation of a polar *u*-¹³C-Algal lyophillized cell (Euroisotop, CLM-2065-05) extract after stereoisomerisation with the AQC-tag for 15 h at 95°C, using a Chiralpak ZWIX(+) column (150 x 4 mm, 3 µm) employing the short gradient UPLC-QTOF-ESI-MS method in positive ionization mode and an injection volume of 2.5 µL. Shown are the extracted ion chromatograms (XICs) for the corresponding *u*-¹³C-amino acids as well as an excerpt of the mass spectrum of the eluted L-enantiomers and D-enantiomers. Enantiomer separation was performed on Chiralpak ZWIX(+) column (150 x 4 mm, 3µm) using the short gradient elution UHPLC-QTOF-ESI-MS method in positive ionisation mode. Note that m/z values highlighted in grey were clearly identified as AQC-tagged *u*-¹³C amino acids, while m/z values in black italic were similar to those of the corresponding AQC-tagged *u*-¹²C amino acids. Also other stereoisomerised amino acid type compounds and non-chiral metabolites were found. Data are not shown. Sample preparation provided in **Example 11.**

### DETAILED DESCRIPTION

The present invention relates to an improved method for stereoisomerization (racemization and epimerization) of compounds that allows for the generation of previously not accessible stereoisomers of chiral compounds, and mixtures thereof, comprising any ratios of stereoisomers. In particular the invention pertains to mixtures of stereoisomers that, previously, have not been obtainable, or were obtainable only using highly laborious methods.

Unless otherwise specified, the terms "include," "includes," "including" and the like are intended to be open-ended. Thus, for example, "include" means "include but are not limited to."

Except as otherwise noted, the articles "a," "an," and "the" mean "one or more."

As used herein, the term "about" encompasses the range of experimental error that occurs in any measurement such as quantifications. Unless otherwise stated, all numerical values with the word "about" in front of them shall specify that the indicated numerical value may vary depending on the usual experimental error. In certain embodiments the term "about" shall refer to a range of +/- 20% of the indicated numerical value, more preferably of +/- 10%, +/-5% and most preferably of +/-2% or less of the indicated value.

The term "alkyl" as employed herein by itself or as part of another group refers to both straight and branched chain saturated radicals of, in some embodiments, up to 50, 40, 30, 20 preferably 10 carbons, unless the chain length is otherwise limited, such as methyl, ethyl, propyl, isopropyl, butyl, pentyl, hexyl, isohexyl, heptyl, octyl, nonyl, decyl and the like.

The invention therefore is defined in a first aspect by a method for stereoisomerizing (racemizing or epimerizing) a chiral compound, the method comprising the process steps:
(a) providing a mixture comprising:
   (aa) the chiral compound which comprises at least one stereogenic carbon atom;
   (bb) a stereoisomerization tag; and
   (cc) optionally, a buffer adjusting the pH of the mixture to a preferred value, such as a basic or acidic pH, more preferably a pH from about **7** to about 10;
   wherein the molar concentration of the racemization tag in the mixture is greater than the molar concentration of the chiral compound; and
(b) heating the mixture to a sufficient temperature for a sufficient time to allow the stereoisomerization (or epimerization) of the chiral compound to take place.

The In some embodiments it is preferred that the buffer is a borate buffer, and even more preferably the molarity of the borate buffer is about 0.4 M and the pH of the borate buffer employed is preferentially about pH 8.

In one preferred embodiment the chiral compound has the following formula (I) 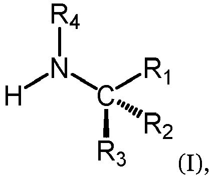 wherein the groups R₁, R₂ and R₃ are chemically not identical, may form a ring, and are independently selected from the group consisting of hydrogen, linear or branched alkyl group, aromatic groups, heteroaromatic groups, carboxyl group, phosphonic acid group, sulfonic acid group, ester group, amide group, carbamate group or urea group, wherein each of R₁, R₂ and R₃ may contain one or more isotopically labelled atoms. Furthermore R₄, C and N may or may not be isotopically labeled. R₄ may be anything, but preferably is also selected from the group consisting of hydrogen, linear or branched alkyl group, aromatic groups, hetero-aromatic groups, carboxyl group, phosphonic acid group, sulfonic acid group, ester group, amide group, carbamate group or urea group, and also may contain one or more isotopic atoms.

The here presented invention in some examples describes the mild N-terminal epimerization of 6-aminoquinolyl-N-hydroxysuccinimidyl (AQC) tagged proteinogenic amino acids, uncommon amino acids and, di- and tripeptides without deamidation of amide bond containing side chains of glutamine, asparagine, theanine and citrulline and without decomposition of tryptophane. Furthermore, this procedure allows the preparation of a CN-isotopically labelled chiral amino acid mixture containing a defined amount of CN-isotopically labelled D-amino acids, which can be used as an internal standard for different sample types, containing varying levels of D-amino acids. In addition, the stereoisomerization (epimerization and racemization) of uncommon amino acids, allo-isoleucine, allo-theonine, theanine, citrulline and hydroxyl-proline as well as the N-terminal epimerization of a dipeptide as well as a tri-peptide are shown. The invention also allows the epimerization of proteinogenic and uncommon amino acids directly in a cell extract, as shown for unified (u)-¹³C,¹⁵N labeled algal lyophilized cells, which thus allows the preparation of a chiral isotopically labeled cell extract applicable as an internal standard for accurate quantification of biological or diagnostic samples containing chiral amino acids and analogs. Furthermore, the invention allows the preparation of chiral degradation and oxidation products of said amino acids and amino acid analogs, as shown for u-¹³C, ¹⁵N-methionine sulfoxide and u-¹³C, ¹⁵N-methionine sulfone.

In some preferred embodiments the stereoisomerization is performed in the presence of acid or base. In other preferred embodiments the mixture comprises an acid or base. In other embodiments the reaction is performed in an inert gas environment (or atmosphere).

The term "stereoisomer" means isomeric molecules that have the same molecular formula and sequence of bonded atoms (constitution), but differ only in the three-dimensional orientations of their atoms in space.

The term "stereoisomerization" or "stereoisomerizing" refers to a process in which a stereoconversion from one stereoisomer into another occurs i.e. in which a stereoisomerically chiral compound is converted from one stereoconfiguration to another different stereoconfiguration.

The term "epimerization" or "epimerizing" as used herein refers to a process in which one out of two or more of the chiral centers in an optically active compound change its configuration.

The term "racemization" or "racemizing" shall refer to a process of stereoisomerization of a chiral compound as described before but with the result of finally obtaining a racemic mixture of two enantiomers (in a ratio 50:50) of the chiral compound.

The term "stereogenic center" shall denote an atom in a chiral compound, bearing groups such that an interchanging of any two groups leads to a stereoisomer.

The term "stereogenic carbon atom" shall refer to a carbon atom in a chiral compound which is a stereogenic center.

The term "enantiomer" means one of two stereoisomers that are mirror images of each other that are non-superimposable (not identical). The group of enantiomers is therefore a subgroup of the larger group of stereoisomers of a chiral compound.

The term "racemic mixture" or "racemate" means a mixture of stereoisomers that has equal amounts of left- and right-handed enantiomers of a chiral molecule with regard to one chiral center, whereas the term "epimeric mixture" refers to a mixture of stereoisomers that has non-equal amounts of left- and right-handed enantiomers of a chiral molecule with regard to one chiral center in the presence of two or more chiral centers. Furthermore the term "stereoisomeric mixture" refers to a mixture of stereoisomers that has equal or non-equal amounts of left- and right-handed enantiomers in the presence of one or more chiral centers.

The term "isomer" means each of two or more compounds with the same chemical bond structure and sum formula, but a different arrangement of atoms in the molecule and different properties. The group of isomers therefore encompasses the more specified groups of stereoisomers and enantiomers as defined above.

The term "diastereomer" denotes a stereoisomer with two or more centers of chirality, wherein diastereomers are such molecules that are not mirror images of one another.

The term "epimer" shall pertain to a molecule that is one of a pair of stereoisomers. The two isomers differ in configuration at only one of two or more stereogenic centers.

The term "alkyl" as used herein is a branched or unbranched saturated hydrocarbon group of 1 to 40 carbon atoms (a smaller range of carbon atoms may be specified herein as "Cx-Cy alkyl" where x and y are integers], such as methyl, ethyl, propyl, isopropyl, n-butyl, isobutyl, t-butyl, pentyl, hexyl, heptyl, octyl, decyl, or tetradecyl, and the like. The alkyl group can be substituted or unsubstituted and should be construed as either if not specified. The term "unsubstituted alkyl group" is defined herein as an alkyl group composed of just carbon and hydrogen. The term "substituted alkyl group" is defined herein as an alkyl group with one or more hydrogen atoms substituted with a group including, but not limited to, an aryl group, a substitute aryl group, a heteroaromatic group, a substituted heteroaromatic group, cycloalkyl group, an alkenyl group, an alkynyl group, an amino group, an amide group, a carbamate group, a urea group, a guanidine group, an ester, an aldehyde, a ketone, a hydroxyl group, an alkoxy group, a thiol group, a thioalkyl group, or a halide, an acyl halide, an acrylate, or a vinyl ether. For example, the alkyl groups can be an alkyl hydroxy group, where any of the hydrogen atoms of the alkyl group are substituted with a hydroxyl group.

The term "alkyl" as defined herein also includes cycloalkyl groups. The term "cycloalkyl group" as used herein is a non-aromatic carbon-based ring composed of at least three carbon atoms, and in some embodiments from three to 20 carbon atoms. Examples of cycloalkyl groups include, but are not limited to, cyclopropyl, cyclobutyl, cyclopentyl, cyclohexyl, etc. The term cycloalkyl group also includes a hetero cycloalkyl group, where at least one of the carbon atoms of the ring is substituted with a heteroatom such as, but not limited to, nitrogen, oxygen, sulfur, or phosphorus.

The term "aromatic group" as used herein denotes any "aryl group" and pertains to any carbon-based aromatic group including, but not limited to, benzene, naphthalene, etc. The term "aryl group" also includes "heteroaromatic groups", also known as "heteroaryl group", which means an aromatic ring composed of at least three carbon atoms that has at least one heteroatom incorporated within the ring of the aromatic group. Examples of heteroatoms include, but are not limited to, nitrogen, oxygen, sulfur, and phosphorus. The aryl group can be substituted or unsubstituted. The aryl group can be substituted with one or more groups including, but not limited to, alkyl, alkynyl, alkenyl, aryl, halide, nitro, amino, ester, ketone, aldehyde, hydroxy, carboxylic acid, or alkoxy as defined herein. In some embodiments, the term "aryl group" is limited to substituted or unsubstituted aryl and heteroaryl rings having from three to 30 carbon atoms.

The term "carboxyl group" refers to an organic functional group consisting of a carbon atom double bonded to an oxygen atom and single bonded to a hydroxyl group. Thus the "carboxyl group" refers to an organic functional group of the formula CO₂H. For the avoidance of doubt, the term "carboxyl group" does not include any other organic functional groups comprising a carbonyl (C=O), such as, for example, aldehydes or ketones.

The term "phosphonic acid group", according to the invention, refers to a chemical group having the following structure: -PO(OH)2. Thus, according to the invention, phosphonic acid groups can be defined as -PO(OH₂) groups which, in a preferred embodiment of the invention, are covalently linked to a carbon atom, e.g. as they replace a hydrogen atom or the carboxylic acid group on a chiral carbon atom or a non-chiral carbon atom (phospho amino acids), or in other words, the -PO(OH2) groups are substituents of aliphatic radicals or groups, alkyl-, alkenyl-, alkynyl-, cycloalkyl- , heterocyclo or aryl-groups, as defined herein.

The term "sulfonic acid group" refers to a sulfonic acid or an alkyl group, as defined herein, having a sulfonic acid group attached thereto.

The terms "ester group", "amide group, "carbamate group" and "urea group" shall be understood according to their general meaning in the art, which is well known to the skilled person.

The heating in step (b) may be performed by using (b.1) heating to a temperature from about 50 to about 200°C for 30 minutes to 24 hours; or (b.2) treating the mixture in a microwave at about 200 to about 400 W for 1 min to 6 h, preferably about 2 to about 120 min, at a temperature from about 50 to about 300°C, more preferably about about 50 to about 150°C. Any mean known to the skilled artisan can be used to heat the mixture, but preferably involves using a heat block or thermos-shaker. More preferably, the heating in step b.1) is at a temperature range from about 55 to about 95°C.

In some preferred embodiments the method according to the invention comprises after the process step a) but before the process step b.1), respectively the process step b.2), a further step of degassing the mixture, preferably by ultrasonification and/or purging with nitrogen, helium or argon and covering the reaction solution with an inert gas, preferably nitrogen or argon.

It shall be understood that the buffer for adjusting the pH of the mixture indicated under step (a) item (cc) above may be either realized by direct addition of a buffer for adjusting the respective pH, or alternatively, the pH may be directly adjusted within the mixture without the use of external buffer media. To adjust the mixture to a pH value below about 8, for example about 7, it is preferable to use a borate buffer, such as an about 0.4 M sodium borate buffer. A preferred embodiment of the invention pertains to an adjustment of the mixture to a pH of about about 8 using an about 0.4 M borate buffer solution.

A "stereoisomerization tag" in context of the present invention shall be any compound that can be used to generate a specific stereoisomer from a mixture of stereoisomers of the chiral compounds, but more preferably the stereoisomerization tag of the invention racemization tag results from or comprises or consists of at least one compound selected from the group consisting of the following: compounds A1 to A9. 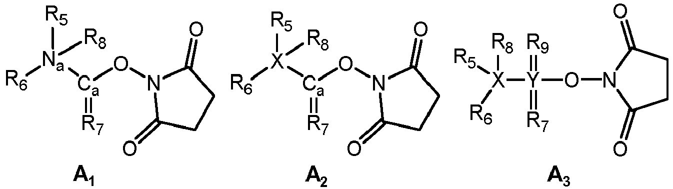 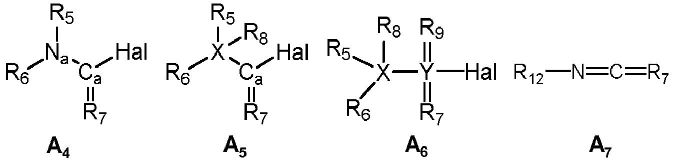 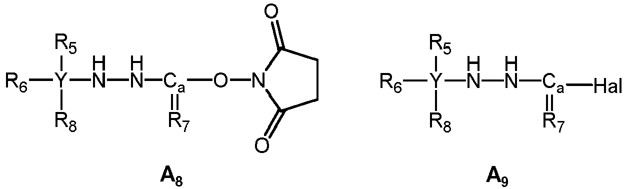
wherein R₅, R₆, R₈ and R₁₂ are independently selected from the group consisting of hydrogen, linear or branched alkyl groups, aromatic groups, heteroaromatic groups, carboxyl group, phosphonic acid group, sulfonic acid group, ester group, amide group, carbamate group or urea group, optionally containing one or more isotopically labelled atoms; and
Hal and OSu are leaving groups
R₇ and R₉ may be independently selected from N, O, S or Se or isotopes thereof;
X may be N, O, S, Se, C or isotopes thereof,
Y may be C, S, P, Se, or isotopes thereof,
wherein in the formulas A1 to A9 furthermore each of the remaining N or C may be ¹⁴N, respectively ¹²C, or isotopes of N and C.

In one preferred embodiment of the invention the racemization tag comprises or consists of one of the compounds A1 and A4 or a mixture thereof.

Most preferred embodiments of the invention pertain to a racemization tag which is selected from aminophenyl-N-hydroxysuccinimidyl carbamate (AC), 3-aminopyridyl N-hydroxysuccinimidyl carbamate (APC) and, most preferably, 6-aminoquinolyl-N-hydroxysuccinimidyl carbamate.

In general the type of chiral compound shall not be restricted in any way. The applicability of the invention for the stereoisomerization (epimerization or racemization) of many different structures is shown in the appended examples. However, it is preferred that the molecules stereoisomerized (epimerized or racemized) according to the invention are preferably selected from the group consisting of amino acids, preferably proteinogenic amino acids, but includes non-proteinogenic, non-ribosomal and synthetic amino acids, as well as amino acid analogues, such as phospho amino acids, and oxidation and degradation products thereof, such as methionine sulfoxide and methionine sulfone. Furthermore the invention includes peptides having a free amino group at the terminal end thereof, proteins having an amino group at the N-terminal end thereof, or mixtures thereof, wherein the respective compounds may comprise at least one isotopically labelled atom.

In a further embodiment it is preferred that the chiral compound comprises heat-, acid- and/or alkaline-labile substituents, such as a sulfonamide group, an amide group, a urea group,a carbamate group or an indole group.

In some embodiments of the invention the mixture further comprises at least one solvent. Suitable solvents for use in the reaction of the invention include solvents in which the chiral compound and the racemization tag are substantially soluble at the reaction temperature. Non-limiting examples of suitable solvents are conventional organic solvents such ethyl acetate, isopropyl acetate, butyl acetate, acetone, acetonitrile, methyl tert-butyl ether (MTBE), tetrahydrofuran, 1,4- dioxane, dimethylsulfoxide, dimethylformamide, diethyl ether and C₁ to C₄ alcohols, toluene, as well as water and mixtures of the foregoing. A particularly preferred solvent is selected from the group consisting of water acetonitrile, methanol, ethanol, propanol, butanol, THF, DMSO, DMF, most preferred water or acetonitrile. In an optional embodiment, which is preferred, the solvent is isotopically labelled, for example using 2H. This embodiment is advantageous because the chiral compound becomes isotopically labelled at its chiral centre during the stereoisomerization (epimerization or racemization) process. In addition, amino acids such as Glu, Asp, Gln, Asn, and Tyr become further labelled by two additional deuterium ions (see also US Patent 3,699,159 (Inventor Irving Putter, Assignee Merck Co Inc; 17 Oct. 1972; both documents are incorporated herein their entireties by reference). Furthermore, employing an isotopically labeled solvent with ¹⁸O provides the additional generation of ¹⁸O-labelled oxidation products of said chiral compounds during the epimerization process, for example Met(¹⁸O) and Met(¹⁸O)₂.

The method of the invention is preferred wherein the matrix solution in which the respective chiral compounds are dissolved is an acidic solution, for example 0.1 M HCl as it is the case, as a mere non limiting example, for a u-¹³C¹⁵N-metabolic amino acid mixture, a buffer solution, for example 0.4 M sodium borate buffer at pH 8.0 or a biological matrix solution, for example a u-¹³C algal lyophilized cell extract solution.

The method of the invention in some embodiments includes that the stereoisomerization (epimerization or racemization) process is performed in the presence of air and/or oxygen, hence generating additional oxidation and degradation products of the respective chiral compounds. In addition oxidation and degradation can be performed in isotopically labeled solvents. The generated oxidation- and degradation products may contain one or more isotopically labeled entities and may or may not have an additional stereogenic center generated. For example, the epimerization of u- ¹³C,¹⁵N-Met alone in solution or as part of a mixture performed in the presence of air and/or oxygen and ²H₂¹⁸O or H₂¹⁸O as part of the solvent leads to the formation of u- ¹³C¹⁵N-Met-S¹⁸O, wherein the sulfur atom has gained chirality during the oxidation process, hence the chiral compound has gained an additional stereogenic center. In addition also u- ¹³C¹⁵N-Met-S¹⁸O₂ may be formed, for which the number of stereogenic centers is not increased after the stereoisomerization (epimerization or racemization) process.

In some embodiments the method for stereoisomerization (epimerization or racemization) may comprise a step of activating the chiral compound to achieve a compound of the following formula (IIa):
(IIa), 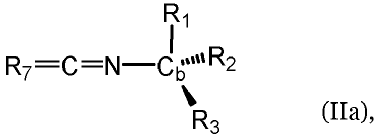 wherein R₇ may be selected from N, O, S or Se or isotopes thereof; or preferably is any of the following compounds (II) and (III): 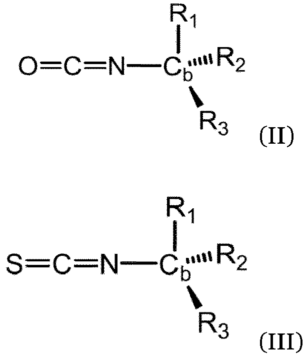 wherein in the method, the stereoisomerization tag comprises at least one of the following compounds 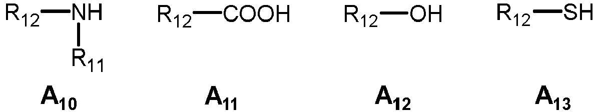 wherein R₁₁ and R₁₂ are independently selected from the group consisting of hydrogen, linear or branched alkyl groups, aromatic groups, heteroaromatic groups, carboxyl group, phosphonic acid group, sulfonic acid group, ester group, amide group, carbamate group or urea group, optionally containing one or more isotopically labelled atoms.

Both strategies, employing an activated stereoisomerization-tag according to the formula A1-A9 as well as employing an activated enantiomer according of the formula IIa or more specific, formula II and/or III combined with an non-activated stereoisomer according to formula A10-A13 provide the same intermediate of formula (IV). Furthermore, the invention pertains to a method comprising the generation of intermediate structure (IV), as well as to the intermediate itself with the above definitions of substituents.

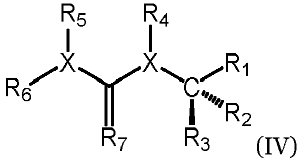

In some embodiments it is further preferable, after completion of the stereoisomerization (epimerization or racemization) process, to remove the stereoisomerization tag from the stereoisomerized (epimerized or racemized) compound. Total cleavage of the stereoisomerization tag from the stereoisomerized (epimerized or racemized) compound can for example be performed by microwave irradiation, or any comparable means, and as a mere non limiting example, microwave irradiation for 5 min at 95°C. Removal of the cleavage product of the stereoisomerization tag can be performed chromatographically via reversed phase chromatography. Chiral separation to obtain purified stereoisomers (or enantiomers) can be performed for the produced stereoisomers carrying the stereoisomerization tag or for products without stereoisomerization tag. In case of complex multiple component mixtures, non-chiral separation of stereoisomers with stereoisomerization tag can be performed via reversed phase chromatography according to the state of the art, whereas non-chiral separation of stereoisomers without stereoisomerization tag can be performed via HILIC chromatography according to the state of the art. Chiral separation of stereoisomers with stereoisomerization tag can be performed with the chiral columns ChiralPAK ZWIX(+), ChiralPAK ZWIX(-), ChiralPAK QNAX, ChiralPAK QDAX. In case of complex mixtures of stereoisomers also a 2D-LC method combining non-chiral and chiral separation can be performed. In case of a surface-bound stereoisomerization tag, as described above, the stereoisomerized (epimerized or racemized) product can be directly obtained in solution, after cleavage of the surface-bound stereoisomerization tag, without additional purification steps.

Yet another embodiment is preferable, wherein (i) the chiral compound and/or (ii) the racemization tag, is isotopically labelled with a stable, meta-stable or unstable isotope. The isotope is preferably selected from ¹¹C, ¹²C, ¹³C, ¹⁴C, ¹⁴N, ¹⁵N, ¹⁶O, ¹⁷O, ¹⁸O, ³²S, ³³S, ³⁴S, ³⁵S, ³⁶S, ⁷⁴Se, ⁷⁶Se, ⁷⁷Se, ⁷⁸Se, ⁷⁹Se, ⁸⁰Se, ⁸²Se, ³¹P, ³²P, ³³P, ¹²³I, ¹²⁷I, ¹²⁹I, ¹³¹I, ¹³⁵I, ¹⁸F, ¹⁹F, ¹H, ²H and ³H, and most preferably an isotope selected from ¹²C, ¹⁴N, ¹⁶O, ³²S, ³¹P, ¹²⁷I, ¹⁹F and ¹H is used.

The method of the invention shall not be restricted to the use of stereoisomerization (epimerization or racemization) of simple chiral compounds, but also includes the possibility to generate stereoisomers of compounds having multiple stereogenic centers. Therefore, in some preferred embodiments, wherein the chiral compound comprises one or more stereogenic centers, such as at least two, at least three, at least four, at least five, or more, stereogenic centers. By targeted addition of the racemization tag to only one of the multiple stereogenic centers it is possible to selectively epimerize such more complex chiral compounds. Another advantage of the method of the present invention.

For the method of the invention it may be preferred that the stereoisomeization-tag is bound to a substrate, such as a particle, a resin, a monolith, a membrane or a plane surface. A typical bead or resin includes a polymer particle, silica gel particle, a nanoparticle, a nanorod and a magnetic bead; or wherein the plane surface includes the inner surface of a wellplate, cartridge, pipette tip, microchip or other tubular structure.

The present invention in another aspect then pertains to a method for producing a (S) stereoisomer with respect to the stereogenic center C of a chiral compound having one or more stereogenic centers according to formula I above, the method comprising performing the method of stereoisomerizing (epimerizing or racemizing) according to the invention, wherein the stereogenic center C of the chiral compound of formula I is in (R) configuration. Thus, the present invention provides a method for the conversion of a (R) stereoisomer into a (S) stereoisomer. For example the conversion could be the conversion of a (R) enantiomer or diastereomer into a (S) enantiomer or diastereomer.

Then in another aspect the invention pertains to a method for producing a (R) stereoisomer with respect to the stereogenic center C of a chiral compound having one or more stereogenic centers according to formula I in claim 1, the method comprising performing the method steps of any one of claims 1 to 15; wherein the stereogenic center C of the chiral compound of formula (I) is in (S) configuration. Thus, the present invention provides a method for the conversion of a (S) stereoisomer into a (R) stereoisomer. For example the conversion could be the conversion of a (S) enantiomer or diastereomer into a (R) enantiomer or diastereomer.

By performing the method of the invention multiple times in sequence, thereby targeting different chiral centers in one compound, it is possible to create virtually any stereoisomer of any given chiral compound irrespective of its complexity.

Another aspect of the invention then pertains to a method of producing a predefined mixture of stereoisomers (or epimers) of a chiral compound according to formula (I) wherein the predefined mixture of stereoisomers (or epimers) comprises a predetermined ratio Y of (R)/(S)-stereoisomers (or epimers) of the stereogenic center C of the chiral compound according to formula (I);
wherein the predefined mixture of stereoisomers (or epimers) is obtained by performing the steps of a method for stereoisomerizing (epimerization or racemization) in accordance to the invention; and
wherein the mixture of step a) in the method according to the invention comprises the chiral compound in a ratio X of (R)/(S) stereoisomers (or epimers) of the stereogenic center C, wherein X < Y; and
wherein the temperature and/or time in step (b.1) or the power (W) and time in step (b.2), is selected to stereoisomerize (epimerize) a sufficient amount of (R) stereoisomer (or epimers) to the (S) stereoisomer (or epimer) to arrive at the predetermined ratio Y of (R)/(S)- stereoisomers (or epimers) in the reaction mixture and to thereby obtain the predefined stereoisomer (epimer or racemic) mixture.

Another aspect of the invention then pertains to a method of producing a predefined mixture of stereoisomer (or epimers) of a chiral compound according to formula (I) wherein the predefined mixture of stereoisomer (or epimers) comprises a predetermined ratio Y of (R)/(S)-stereoisomers (or epimers) of the stereogenic center C of the chiral compound according to formula (I);
wherein the predefined mixture of stereoisomers (or epimers) is obtained by performing the steps of a method for stereoisomerization (epimerization or racemization) of the invention;
wherein the mixture of step a) in the method according to the invention comprises the chiral compound in a ratio X of (R)/(S)epimers of the stereogenic center C, wherein X > Y; and
wherein the temperature and/or time in step (b.1) or the power (W) and time in step (b.2), is selected to stereoisomerize a sufficient amount of (R) stereoisomer (or epimers) to the (S) stereoisomer (or epimers) to arrive at the predetermined ratio Y of (R)/(S)-stereoisomer in the reaction mixture and to thereby obtain the predefined racemic mixture. In some preferred embodiments the epimers are enantiomers, in other embodiments the stereoisomers (or epimers) are diastereomers of each other.

Preferably the methods as described herein are used to produce a predefined mixture of stereoisomers (or epimers) for use as an internal metabolite control (standard), for example in mass spectrometry. The mixture of stereoisomers is in some embodiments a non-racemic mixture or alternatively is a racemic mixture.

Yet another aspect provides a predefined mixture of stereoisomers of a chiral compound, wherein the mixture of stereoisomers is obtainable by a method of producing a predefined mixture of epimers as described herein before.

Furthermore provided is a use of the predefined mixture of stereoisomers (enantiomers or diastereomers) according to the invention in a method of detecting and/or quantifying one or more chiral biological compounds in a biological sample. The predefined mixture of stereoisomers is used herein as an internal standard in the method of detecting, identifying and/or quantifying.

The above methods for generating a predefined mixture of epimers may also be employed to generate a predefined mixture of stereoisomers which are not epimers, which therefore differ from each other in their configuration at more than one chiral center of the compound. Such more complex mixtures are however easily obtainable by a method of the invention by simply performing the method of stereoisomerization (or epimerization) according to the invention multiple times and/or in the presence of oxygen. For example to generate first an stereoisomer at a first chiral center, and then using the so generated stereoisomers, to generate therefrom a second stereoisomer with respect to another chiral center (which does not equal the first chiral center) and thereby generate a complex stereoisomer compound. The first educt and the final product of this multi-step process are therefore stereoisomers of each other, but are different in configuration at more than one chiral center, and thus are not epimers of each other. For example, to generate first an stereoisomer (or epimer) at a first chiral center, and then performing the epimerization in the presence of oxygen using the so generated stereoisomer (or epimer), to generate therefrom a second stereoisomer (or epimer) by generation of another stereogenic center (which does not equal the first stereogenic center). Such would happen during oxidative stereoisomerization (epimerization) of methionine to methionine-sulfoxide, hence generating a second chiral center at the sulfur atom.

In some embodiments it may be preferred that the predefined mixture of stereoisomers (or epimers) is an isotope labelled internal standard. Such standards in particular are useful in methods for quantification of naturally occurring stereoisomeric compounds, for example using mass spectrometry. The method of detecting, identifying and/or quantifying in accordance with the invention is preferably an imaging based technology, a nuclear magnetic resonance (NMR) method, or is a mass spectrometry (MS) method, preferably is an MS method ionization selected from atmospheric chemical ionization (APCI), atmospheric pressure photoionization (APPI), electrospray ionization (ESI), inductively coupled plasma ionization (ICP), chemical ionization (CI), electron impact (EI), fast atom bombardment (FAB), field desorption/field ionization (FD/FI), thermospray ionization (TSP) and matrix-assisted laser desorption (MALDI), hyphenated with various mass analyzers such as single-quadrupole, triple-quadrupol-MS (TripleQuad-MS), ion-trap (IT-MS), orbitrap-based MS, triple quadrupole linear ion traps (QTRAP-MS), time-of-flight MS (TOF-MS), quadrupole time-of-flight MS (QTOF-MS), fourier transform ion cyclotron mass spectrometry (FT-ICR-MS),

Another aspect of the invention provides a method of detecting, identifying and/or quantifying (R)-and/or (S)-stereoisomers of a chiral compound (analyte) in a biological sample from a subject, comprising: (a) preparing a sample by adding a predefined mixture of stereoisomers as described before, as internal standard, into the biological sample; (b) analyzing the eluent for the presence of said stereoisomers, for example using a mass analyzer. In preferred embodiments the stereoisomers are enantiomers, epimers, or diastereomers.

In accordance with the herein disclosed invention, a biological sample may be selected from any non-cellular or cellular sample comprising at least one biomolecule. Preferred are cellular samples, for example samples comprising any prokaryotic or eukaryotic cells, such as bacteria, archaea, uni- or multi-cellular eukaryotic cells, such as fungi, plant cells, insect cells or animal cells. More preferably the sample is a liquid ample such as a homogenized tissue sample or is a bodily fluid selected from the group consisting of saliva, sweat, urine, blood, serum, plasma, spinal fluid, and combinations thereof. In preferred embodiments the biological sample is a mammalian sample, such as a mouse, rat or human sample. Most preferred are human samples obtained from a patient.

A method for quantification in accordance with the invention may preferably be for use in diagnosing a disorder in the subject such as a human patient, preferably wherein the disorder is characterized by the presence or absence of a quantity of a stereoisomer in a sample of the subject. The predefined mixtures of stereoisomers as produced in accordance with the invention are advantageously used as internal standard during the quantification of the stereoisomer or ratio of stereoisomers that is associated with the disease to be diagnosed. In this context a first kit containing a solution with a mixture of isotopically labelled and/or non-isotopically labelled proteinogenic amino acid and/or uncommon amino acids and/or peptides and/or proteins and/or cell extract (e.g. algal, yeast, mammalian, bacterial) and/or homogenized tissue sample and or bodily fluids (e.g. plasma), the buffer solution with preferred pH 8 and molarity 0.4 M, stereoisomerization tag and a solvent preferably acetonitrile (to solubilize the stereoisomerization tag). A second kit containing standard solutions with various stereoisomer ratio ranges from 0.1 to 50% of respective stereoisomer of proteinogenic amino acids tagged with the stereoisomerization tag with preferred stereoisomer ratio range 0.5 - 5%, 5-25% and 25-50% of stereoisomers generated by the present invention. A third kit 3 containing standard solutions with various stereoisomer ratio ranges from 0.1 to 50% of respective stereoisomer of proteinogenic amino acids without the stereoisomerization tag with preferred stereoisomer ratio range 0.5 - 5%, 5-25% and 25-50% of stereoisomers generated by the present invention.

In another embodiment a stereoisomer obtainable by a method described herein also forms part of the present invention.

In context of the invention the inventors for the first time produced a wide variety of stereoisomers which were previously not obtainable by isolation or any prior art methodology. Therefore, the present invention in another aspect provides at least the following stereoisomers.

The following additional compounds form also part of the invention:

Proteinogenic Amino Acids: D-¹³C₅,¹⁵N-Valine, D-¹³C₆-Leucine, D-¹³C₆,¹⁵N-Leucine, D-¹³C₆-Isoleucine, D-¹³C₆,¹⁵N-Isoleucine, DL-¹³C₆-Isoleucine, DL-¹³C₆,¹⁵N-Isoleucine, D-¹³C₅-Methionine, D-¹³C₅,¹⁵N-Methionine, DL-¹³C₅-Methionine, DL-¹³C₅,¹⁵N-Methionine, D-¹³C₅-Proline, D-¹³C₅,¹⁵N-Proline, DL-¹³C₅-Proline, D-¹³C₃-Serine, D-¹³C3,¹⁵N-Serine, D-¹³C₄-Threonine, D-¹³C₄,¹⁵N-Threonine, DL-¹³C₄-Threonine, DL-¹³C₄,¹⁵N-Threonine, D-¹³C₃-Cystein, D-¹³C₃,¹⁵N-Cystein, D-¹³C₆-Cystine, D-¹³C₆,¹⁵N-Cystine, D-¹³C₉-Phenylalanine, DL-¹³C₉-Tyrosine, DL-¹³C₉,¹⁵N-Tyrosine, D-¹³C₁₁-Tryptophane, D-¹³C₁₁,¹⁵N₂-Tryptophane, DL-¹³C₁₁-Tryptophane, DL-¹³C₁₁, ¹⁵N₂-Tryptophane, DL-¹³C₅,¹⁵N-Glutamic acid, D-¹³C₄,¹⁵N-Aspartic acid, DL-¹³C₄,¹⁵N-Aspartic acid, D-¹³C₅-Glutamine, DL-¹³C₅-Glutamine, DL-¹³C₅,¹⁵N-Glutamine, DL-¹³C₅,¹⁵N₂-Glutamine, D-¹³C₄,¹⁵N₂-Asparagine, DL-¹³C₄-Asparagine, DL-¹³C₄,¹⁵N-Asparagine, DL-¹³C₄,¹⁵N₂-Asparagine, D-¹³C₆-Histidine, D-¹³C₆,¹⁵N-Histidine, D-¹³C₆,¹⁵N₂-Histidine, D-¹³C₆,¹⁵N₃-Histidine, DL-¹³C₆-Histidine, DL-¹³C₆,¹⁵N-Histidine, DL-¹³C₆,¹⁵N₂-Histidine, DL-¹³C₆,¹⁵N₃-Histidine, D-¹³C₆,¹⁵N-Lysine, D-¹³C₆,¹⁵N₂-Lysine, DL-¹³C₆,¹⁵N-Lysine, DL-¹³C₆,¹⁵N₂-Lysine, D - ¹³C₆-Arginine, D-¹³C₆,¹⁵N-Arginine, D-¹³C₆,¹⁵N₂-Arginine, D-¹³C₆,¹⁵N₃-Arginine, D-¹³C₆,¹⁵N₄-Arginine, DL-¹³C₆-Arginine, DL-¹³C₆,¹⁵N-Arginine, DL-¹³C₆,¹⁵N₂-Arginine, DL-¹³C₆,¹⁵N₃-Arginine,

Oxidation Products from Met: L-¹³C₅,¹⁵N-Methionine sulfoxide (S-(*S*)), L-¹³C₅,¹⁵N-Methionine sulfoxide (S-(*R*)), D-¹³C₅,¹⁵N-Methionine sulfoxide (R-(*S*)), D-¹³C₅,¹⁵N-Methionine sulfoxide (R-(*R*)), DL-¹³C₅,¹⁵N-Methionine sulfoxide (RS-(*S*)), DL-¹³C₅,¹⁵N-Methionine sulfoxide (RS-(*R*)), L-¹³C₅,¹⁵N,¹⁸O-Methionine sulfoxide (S-(*S*)), L-¹³C₅,¹⁵N,¹⁸O-Methionine sulfoxide (S-(*R*)), D-¹³C₅,¹⁵N,¹⁸O-Methionine sulfoxide (R-(*S*)), D-¹³C₅,¹⁵N,¹⁸O-Methionine sulfoxide (R-(*R*)), DL-¹³C₅,¹⁵N,¹⁸O-Methionine sulfoxide (RS-(*S*)), DL-¹³C₅,¹⁵N,¹⁸O-Methionine sulfoxide (RS-(*R*)), L-¹³C₅,¹⁵N-Methionine sulfone (S), D-¹³C₅,¹⁵N-Methionine sulfone (R), DL-¹³C₅,¹⁵N-Methionine sulfone (RS), L-¹³C₅,¹⁵N,¹⁸O-Methionine sulfone (S), D-¹³C₅,¹⁵N,¹⁸O-Methionine sulfone (R), DL-¹³C₅,¹⁵N,¹⁸O-Methionine sulfone (RS), L-¹³C₅,¹⁵N,¹⁸O₂-Methionine sulfone (S), D-¹³C₅,¹⁵N,¹⁸O₂-Methionine sulfone (R), DL-¹³C₅,¹⁵N,¹⁸O₂-Methionine sulfone (RS), L-¹³C₅-Methionine sulfoxide (S-(*S*)), L-¹³C₅-Methionine sulfoxide (S-(*R*)), D-¹³C₅-Methionine sulfoxide (R-(*S*)), D-¹³C₅-Methionine sulfoxide (R-(*R*)), DL-¹³C₅-Methionine sulfoxide (RS-(*S*)), DL-¹³C₅-Methionine sulfoxide (RS-(*R*)), L-¹³C₅,¹⁸O-Methionine sulfoxide (S-(*S*)), L-¹³C₅,¹⁸O-Methionine sulfoxide (S-(*R*)), D-¹³C₅,¹⁸O-Methionine sulfoxide (R-(*S*)), D-¹³C₅,¹⁸O-Methionine sulfoxide (R-(*R*)), DL-¹³C₅,¹⁸O-Methionine sulfoxide (RS-(*S*)), DL-¹³C₅,¹⁸O-Methionine sulfoxide (RS-(*R*)), L-¹³C₅-Methionine sulfone (S), D-¹³C₅-Methionine sulfone (R), DL-¹³C₅-Methionine sulfone (RS), L-¹³C₅,¹⁸O-Methionine sulfone (S), D-¹³C₅,¹⁸O-Methionine sulfone (R), DL-¹³C₅,¹⁸O-Methionine sulfone (RS), L-¹³C₅,¹⁸O₂-Methionine sulfone (S), D-¹³C₅,¹⁸O₂-Methionine sulfone (R), DL-¹³C₅,¹⁸O₂-Methionine sulfone (RS).

Decomposition Product from Trp: L-¹³C₁₀,¹⁵N₂-Kynurenine, D-¹³C₁₀,¹⁵N₂-Kynurenine, DL-¹³C₁₀,¹⁵N₂-Kynurenine, L-¹³C₁₀,¹⁵N-Kynurenine, D-¹³C₁₀,¹⁵N-Kynurenine, DL-¹³C₁₀,¹⁵N-Kynurenine, L-¹³C₁₀-Kynurenine, D-¹³C₁₀-Kynurenine, DL-¹³C₁₀-Kynurenine

Uncommon Amino Acids: D-¹³C₆-allo-Isoleucine, D-¹³C₆,¹⁵N-allo-Isoleucine, DL-¹³C₆-allo-Isoleucine, DL-¹³C₆,¹⁵N-allo-Isoleucine, D-¹³C₄-allo-Threonine, D-¹³C₄¹⁵N-allo-Threonine, DL-¹³C₄-allo-Threonine, DL-¹³C₄¹⁵N-allo-Threonine, D-¹³C₄-Homoserine, D-¹³C₄¹⁵N-Homoserine, DL-¹³C₄-Homoserine, DL-¹³C₄¹⁵N-Homoserine, D-¹³C₄,¹⁵N-Homocystein, D-¹³C₄-Homocystein, DL-¹³C₄,¹⁵N-Homocystein, DL-¹³C₄-Homocystein, D-¹³C₈¹⁵N₂-Homocystine, D-¹³C₈¹⁵N-Homocystine, D-¹³C₈-Homocystine, DL-¹³C₈N₂-Homocystine, DL-¹³C₈N-Homocystine, DL-¹³C₈-Homocystine, D-¹³C₅,¹⁵N₂-Ornithine, D-¹³C₅,¹⁵N-Ornithine, D-¹³C₅-Ornithine, DL-¹³C₅,¹⁵N₂-Ornithine, DL-¹³C₅,¹⁵N-Ornithine, DL-¹³C₅-Ornithine, D-¹³C₆,¹⁵N₃-Citrulline, D-¹³C₆,¹⁵N₂-Citrulline, D-¹³C₆,¹⁵N-Citrulline, D-¹³C₆-Citrulline, DL-¹³C₆,¹⁵N₃-Citrulline, DL-¹³C₆,¹⁵N₂-Citrulline, DL-¹³C₆,¹⁵N-Citrulline, DL-¹³C₆-Citrulline, D-¹³C₅,¹⁵N-Norvaline, D-¹³C₅-Norvaline, DL-¹³C₅,¹⁵N-Norvaline, DL-¹³C₅-Norvaline, D-¹³C₆,¹⁵N-Norleucine, D-¹³C₆-Norleucine, DL-¹³C₆,¹⁵N-Norleucine, DL-¹³C₆-Norleucine, D-¹³C₄,¹⁵N-2-Aminobutyric acid, D-¹³C₄-2-Aminobutyric acid, DL-¹³C₄,¹⁵N-2-Aminobutyric acid, DL-¹³C₄-2-Aminobutyric acid, D-¹³C₄,¹⁵N-Aminoisobutyric acid, D-¹³C₄-Aminoisobutyric acid, DL-¹³C₄,¹⁵N-Aminoisobutyric acid, DL-¹³C₄-Aminoisobutyric acid, D-¹³C₁₀,¹⁵N₃-Hypusine *(R),* D-¹³C₁₀,¹⁵N₃-Hypusine *(S),* D-¹³C₁₀,¹⁵N₂-Hypusine *(R),* D-¹³C₁₀,¹⁵N₂-Hypusine *(S),* D-¹³C₁₀,¹⁵N-Hypusine *(R),* D-¹³C₁₀,¹⁵N-Hypusine *(S),* DL-¹³C₁₀,¹⁵N₃-Hypusine (R), DL-¹³C₁₀,¹⁵N₃-Hypusine (S), DL-¹³C₁₀,¹⁵N₂-Hypusine (R), DL-¹³C₁₀,¹⁵N₂-Hypusine (S), DL-¹³C₁₀,¹⁵N-Hypusine (R), DL-¹³C₁₀,¹⁵N-Hypusine (S), D-¹³C₁₀,¹⁵N₃-Deoxyhypusine, D-¹³C₁₀,¹⁵N₂-Deoxyhypusine, D-¹³C₁₀,¹⁵N-Deoxyhypusine, D-¹³C₁₀-Deoxyhypusine, DL-¹³C₁₀,¹⁵N₃-Deoxyhypusine, DL-¹³C₁₀,¹⁵N₂-Deoxyhypusine, DL-¹³C₁₀,¹⁵N-Deoxyhypusine, DL-¹³C₁₀-Deoxyhypusine.

In some preferred embodiments the stereoisomer of the invention may be isotopically labelled as described herein elsewhere. In another embodiment there is provided a epimeric, racemic or non-racemic composition comprising a stereoisomer of the invention (table above and/or the above compounds), with or without a racemization tag.

Also provided are pharmaceutical compositions, comprising a stereoisomer or any stereoisomer mixture as described herein together with a pharmaceutically acceptable carrier and/or excipient.

By way of example, the pharmaceutical composition of the invention may comprise between 0.1% and 100% (w/w) active ingredient (herein a stereoisomer of the invention or an inventive mixture of stereoisomers obtained by a method as described herein), such as about 0.5%, 1%, 2%, 3%, 4%, 5%, 6%, 8% 10%, 15%, 20%, 25%, 30%, 40%, 50%, 60%, 70%, 80%, 90%, 95%, 96%, 97%, 98% or 99%, preferably between about 1% and about 20%, between about 10% and about 85% (w/w) agents, such as lubricants, controlled release vehicles, diluents, emulsifying agents, humectants, dispersion media, coatings, antibacterial or antifungal agents, isotonic and absorption delaying agents, and the like, compatible with pharmaceutical administration. The use of such media and agents for pharmaceutically active substances is well-known in the art. Except insofar as any conventional media or agent is incompatible with the active compound, use thereof in the compositions is contemplated. Supplementary agents can also be incorporated into the compositions.

The pharmaceutical composition of (or for use with) the invention is, typically, formulated to be compatible with its intended route of administration. Examples of routes of administration include oral, parenteral, e.g., intrathecal, intra-arterial, intravenous, intradermal, subcutaneous, oral, transdermal (topical) and transmucosal administration.

In certain embodiments, the pharmaceutical composition is formulated for sustained or controlled release of a compound of (or for use with) the invention. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially (including liposomes targeted to infected cells with monoclonal antibodies to viral antigens) can also be used as pharmaceutically acceptable carriers. These can be prepared according to methods known to those skilled in the art.

It is especially advantageous to formulate oral, rectal or parenteral compositions in dosage unit form for ease of administration and uniformity of dosage. Dosage unit form as used herein includes physically discrete units suited as unitary dosages for the subject to be treated; each unit containing a predetermined quantity of active compound calculated to produce the desired therapeutic effect in association with the required pharmaceutical carrier. The specification for the dosage unit forms of the invention are dictated by and directly dependent on the unique characteristics of the active compound and the particular therapeutic effect to be achieved, and the limitations inherent in the art of compounding such an active compound for the treatment of individuals.

Exemplary unit dosage forms for pharmaceutical compositions of the invention are tablets, capsules (eg as powder, granules, microtablets or micropellets), suspensions or as single-use pre-loaded syringes. In certain embodiments, kits are provided for producing a single-dose administration unit. The kit can contain both a first container having a dried active ingredient and a second container having an aqueous formulation. Alternatively, the kit can contain single and multi-chambered pre-loaded syringes.

### EXPERIMENTAL EXAMPLES

The invention is further illustrated by the following non-limiting Examples.

### Example 1: Preparation and stereoisomerisation of AQC-tagged threonine.

The test solutions 0.25 mM L-Thr, L-aThr, DL-Thr and DL-aThr (10 µL) were derivatized using 20 mM borate buffer (80 µL) and 10 µL AQC (3 mg/mL in ACN), and were stereoisomerised for 18 h at 95°C using a thermoshaker. Extracted ion chromatograms are shown in **Fig.ic.**

### Example 2: Comparison of different stereoisomerization tags (APC, AC and AQC as well as different heating methods (thermoshaker and microwave)

After derivatisation of an amino acid solution containing 0.25 mM *u*-¹²C¹⁴N-L-amino acid mixture and 0.25 mM *u*-¹³C¹⁵N-L-amino acid mixture with the stereoisomerisation tags 3-aminopyridyl N-hydroxysuccinimidyl carbamate (APC), aminophenyl-N-hydroxysuccinimidyl carbamate (AC) and 6-aminoquinolyl-N-hydroxysuccinimidyl carbamate solution (AQC) (10 µL sample, 80 µL 20 mM borate buffer and 10 µL stereoisomerisation tag (3 mg/mL in ACN) heated for 10 min at 55°C), stereoisomerisation was performed for the APC and the AC-tagged samples at 95°C for 18 hours using a thermoshaker (TS), and for the AQC-tagged sample at 75°C for 15 min using microwave irradiation (MW). All samples were re-derivatised with 10 µL of the respective stereoisomerisation tag prior to analysis. Extracted ion chromatograms are shown in **Fig.2** **a.**

### Example 3: Stereoisomerization of L-citrulline, L-theanine and trans-4-L-hydroxy-proline

After derivatisation of 0.25 mM solutions of uncommon amino acids, L-citrulline, L-theanine and trans-4-L-hydroxy-proline with 6-aminoquinolyl-N-hydroxysuccinimidyl carbamate solution (AQC) (10 µL sample, 80 µL 20 mM borate buffer and 10 µL AQC (3 mg/mL in ACN) heated for 10 min at 55°C), stereoisomerisation was performed at 95°C for 18h using a thermoshaker. All samples were re-derivatised with 10 µL AQC prior to analysis. Extracted ion chromatograms are shown in **Fig.2** **b.**

### Example 4: Stereoisomerization of the tri-peptide (L-Arg)-Gly-(L-Asp)

To 70 µL 0.2 M borate buffer, 10 µL 2.5 mM (L-Arg)-Gly-(L-Asp) and 20 µL AQC (3 mg/mL in ACN) was added and heated to 55°C for 10 min. The microcentrifuge tube was centrifiged, ultrasonicated for 1 min and covered with nitrogen gas (30 sec). Stereoisomerisation was performed for 15 hours at 95°C using a thermoshaker. In parallel, the same derivatisaton procedure was performed using 0.2 M deuerated borate buffer to induce a hydrogen-deuterium exchange (HDX) at the chiral center. After stereoisomerisation and re-derivatisation with 20 µL AQC the volume was adjusted to 100 µL with ACN. Extracted ion chromatograms shown in **Fig.2c****.**

### Example 5: Influence of in-solution pH values on stereoisomerization results

pH-Values were determined with a micro pH electrode (HI1083B from Hanna Instruments Inc, Voehringen, Germany) for n=4 sample solutions (error bars in red); before derivatisation with 6-aminoquinolyl-N-hydroxysuccinimidyl carbamate (AQC) (n=5), after derivatisation with AQC and after stereoisomerisation at 95°C for 15h. To 180 µL 0.2 M or 0.4 M sodium borate buffer at pH values of 8.8, 8.5, 8.0, 7.5 and 7.0, 10 µL 2.5 mM *u*-¹²C, ¹⁴N-L-amino acid (mixture of all 20 amino acids) solution in 0.1 M HCl (0.50 µmol) was added, followed by derivatisation with 60 µL AQC-solution (3 mg/mL in acetonitrile; 0.63 µmol). Prior to stereoisomerisation at 95°C for 15h, all solutions were ultrasonicated for 1 minute and coated with nitrogen for 1 minute. Enantiomer separation was performed with the ChiralPAK ZWIX (+) (150 x 4 mm, 3 µm) column using the short gradient elution method. Extracted ion chromatograms shown in **Fig.3a****.**

### Experiment 6: Influence of pH on degradation of AQC-tagged glutamine

Stereoisomerisation of AQC-tagged glutamine. Prior to derivatisation, a 2.5 mM L-glutamine stock solution (in 0.1 M HCl) was diluted to 0.25 mM using 10 mM HCl, ultra-pure water and 0.2 M borate buffer (pH 8.83) followed by ultrasonication and replacing air by nitrogen. The pH measurements (n=10) were performed after derivatisation with AQC (10 µL 0.25 mM Gln, 70 µL 0.2 M borate buffer and 20 µL 3 mg/mL AQC in acetonitrile; 10 min at 55°C), before and after stereoisomerisation at 95°C for 95°C using a thermoshaker. Extracted ion chromatograms shown in **Fig.3b****.**

### Example 7: Time and temperature dependent stereoisomerisation Part 1:

To 80 µL 20 mM borate buffer (pH 8.8), 10 µL L-amino acid standard solution, containing 0.025 mM *u*-¹²C¹⁴N amino acids and 0.025 mM *u*-¹³C¹⁵N amino acids and 10 µL 6-aminoquinolyl-N-hydroxysuccinimidyl carbamate (AQC, 3 mg/mL in ACN) were added in a 1.5 mL Safe-Lock reaction tubes from Eppendorf (Hamburg, Germany). The following stereoisomerisation conditions were investigated (n=1), 10 min (55°C, 75°C and 95°C), 30 min (55°C, 75°C and 95°C) and 1 h (55°C, 75°C and 95°C). The shaker speed of a Grant-bio PHMT Thermo-Shaker from Grant Instruments Ltd. (Shepreth, England) was set to 600 rpm. After reaching room temperature, all samples were centrifuged, volume adjusted to 100 µL with ACN and analyzed without further treatment. Enantiomer separation was performed on Chiralpak ZWIX(+) column (150 x 4 mm, 3 µm) using the long UHPLC-QTOF-ESI(+)-MS method. Results are shown in **Fig. 4a** and **Fig. 4b****.**

### Example 8: Time and temperature dependent stereoisomerisation Part 2 and NMR solvent study:

To 80 µL 20 mM borate buffer (pH 8.8), 10 µL L-amino acid standard solution, containing 0.25 mM *u*-¹²C¹⁴N amino acids and 0.25 mM *u*-¹³C¹⁵N amino acids and 10 µL 6-aminoquinolyl-N-hydroxysuccinimidyl carbamate (AQC, 3 mg/mL in ACN) were added in a 1.5 mL Safe-Lock reaction tubes from Eppendorf (Hamburg, Germany). Derivatization was performed at 55°C for 10 min, followed by centrifugation, 1 min ultrasonication and replacement of air with nitrogen (30 sec.). The following stereoisomerisation conditions were investigated (n=3), 1 h, 6 h and 15 h at 95°C and 600 rpm. All samples were centrifuged after reaching room temperature, volume was adjusted to 100 µL and additionally derivatised with AQC (samples (A)), which resembles a 1:10 dilution of the sample to 0.025 mM amino acids. Additionally the 15 h sample was only diluted 1:10 with 0.2 M borate buffer, without additional re-derivatisation (samples (B)). Results are shown in **Fig. 4c** and **Fig. 4d****.**

A saturated L-phenylglycine (PG) solution (50 mg/mL; 15 µL) in acetonitrile:water (1:2, (v/v)) was derivatized with equimolar amount of 6-aminoquinolyl-N-hydroxysuccinimidyl carbamate (AQC, 3 mg/mL in ACN; 478 µL) for 15 min at 55°C. Purification of PG-AQC was performed via C18 reversed phase chromatography on an Eclipse XDB-C18 (4.6 x 150 mm, 5 µm) column from Agilent using an Agilent 1100 HPLC system with binary pump, autosampler, column oven and faction collector, employing 0.01% formic acid in water as mobile phase A (MPA) and HPLC-grade acetonitrile as mobile phase B (MPB). Injection volume was 50 µL. The collected PG-AQC peak fraction was concentrated on a GeneVac. The purified L-PG-AQC was dissolved in ACN-d3:D2O (1:2) and kept as a saturated solution. For the following experiments 100 µL aliquots of this saturated L-PD-AQC solution was used. In case of a change of solvent, 100 µL L-PG-AQC was reduced to dryness in the GeneVac and resuspended in the respective solvent or solution.

a) Purified L-PG-AQC in ACN-d₃:D₂O (1:2) without treatment; samples b) - h) were heated to 95°C for 12 h and differ in their solvent composition: b) L-PG-AQC in ACN-d₃:D₂O (1:2), c) in ACN-d₃:D₂O (1:2) with 0.2 M Na-borate-d₃ buffer (pH 8.8), d) in ACN-d₃:D₂O (1:2) with 0.5% FA, e) in DMSO-d₆, f) in DMSO-d₆ with 0.2 M Na-borate-d₃ buffer (pH 8.8), g) in DMSO-d₆ with 4x10⁻³% NaOD (w/v) and h) in DMSO-d₆ with 4x10⁻⁵% NaOD (w/v).

Chiral separation of phenylglycine-AQC (PG-AQC) was performed on a ChiralPAK ZWIX (+) column (150 x 4 mm, 3 µm) using an UHPLC-QTOF-ESI(+) system. As mobile phase MeOH:H₂O (98:2) with 9.4 mM NH₄Fa and 9.4 mM FA was used at a flow rate of 300 µL/min and 30°C column temperature. Stereoisomerization results were visualized via extracted ion chromatograms (XICs) of [PG-AQC+H]⁺ and [PG-AQC-[²H)+H]+ as well as the mass spectra for D-PG-AQC at 11.6 min and L-PG-AQC at 12.5 min for the HDX-experiments under investigated stereoisomerization conditions.

In general, in the absence of acid or base (e.g. example a) and e)) no stereoisomerization was observed. In the presence of acid such as formic acid and base such as sodium hydroxide stereoisomerization was observed. Concerning solvent type, stereoisomerization was observed in aqueous hydro-organic solutions as well as organic solvents such as DMSO. The stereoisomerization results are: b and h < f and g < c and d.

### Example 9: Stereoisomerisation of an isotopically labelled u-¹³C¹⁵N-L-amino acid mixture

Stereoisomerisation of a solution containing 0.025 mM *u*-¹²C¹⁴N-L-amino acids (20 natural amino acids; grey) and 0.025 mM *u*-¹³C¹⁵N-L-amino acids (Metabolomics Amino Acid Mix from Euroisotop, unlabelled standard without Asn, Gln and Trp from Eurisotop; black) derivatised with 6-aminoquinolyl-N-hydroxysuccinimidyl carbamate (AQC, 3 mg/mL ACN) and incubated for 6 h at 95°C, omitting Cys due to inconsistencies in retention times and too low peak areas. Results are shown in **Fig. 5****.**

### Example 10: Preparation of isotopically labelled u-¹³C¹⁵N-DL-methionine sulfoxide (Met-(O)) and u-¹³C₁₅N-DL-methionine sulfone (Met-(O)2

Preparation of the oxidation products *u*-¹³C¹⁵N-methionine sulfoxide (Met-(O)) and *u*-¹³C¹⁵N-methionine sulfone (Met-(O)₂ from AQC-tagged *u*-¹³C¹⁵N-methionine (present in a 0.025 mM *u*-¹³C¹⁵N- and *u*-¹²C¹⁴N-amino acid mixture) during stereoisomerisation in the presence of oxygen at 95°C for a) 6 h and b) 24 h (0.25 mM *u*-¹³C¹⁵N amino acid mixture); and for c) 3 h and 12 h after ultrasonication and purging with nitrogen. Enantiomer separation was performed after additional derivatisation with AQC. Results are shown in **Fig. 6****.**

### Example 11: Preparation of a chiral isotopically labelled u-¹³C-algal cell extract

10.33 mg *u*-¹³C-algal lyophillized cells (Euroisotop, CLM-2065-05) were weighed into a 1.5 mL microcentrifuge tube, suspended with 50 µL acetonitrile:water (ACN:H2O; 1:1 (v/v)), incubated on ice for 5 min, ultrasonicated for 5 min on ice and homogenized with an Eppendorf Pestile on ice, followed by addition of 950 µL ACN:H₂O (1:1 (v/v)), vortexing for 1 min and centrifugation 3 x 30 sec (turn tube 180°) at 3000 rpm at 4°C. The supernatant was transferred to a 2 mL microcentrifuge tube and extracted with 300 µL TBME (1 min vortexing and 1 min ultrasonication). The top layer, which contained lipids and chlorophyll was transferred to a separate tube and stored at -20°C. The cell pellet was extracted three more time as previously described. The aqueous phase of four extractions were reduced to dryness in a GeneVac, pooled and each was resuspended in 100 µL ACN:H2O; 1:4 (v/v) by 5 min ultrasonication on ice and 1 min vortexing. The *u*-¹³C-Algal extract was stored at -20°C.

After centrifugation, 10 µL of the polar extract was added to 60 µL 0.4 M borate buffer (pH 8.00) followed by the addition of 30 µL AQC (3 mg/mL in ACN). The reaction solution was immediately placed on the thermoshaker at 55°C and 800 rpm for 10 min. The AQC derivatised extract solution was centrifuged, ultrasonicated for 1 min, purged with nitrogen gas for 30 sec and the reaction tube was additionally sealed with Parafilm. The stereoisomerisation reaction was performed on a thermoshaker for 15 h at 95°C. After reaching room temperature, 20 µL AQC (3 mg/mL in ACN) was added and the extract was heated to 55°C for 10 min, in order to re-derivatise amino groups which had lost their AQC-tag during the stereoisomerisation process. The volume of the reaction solution was adjusted to 100 µL with acetonitrile prior to UPLC-QTOF-ESI-MS analysis on a ZWIX (+) column (150 x 4 mm, 3 µm) employing the short gradient elution method for enantiomer separation in positive ionisation mode. Results are shown in **Fig. 7****.**

### Example 12: Stereoisomerisation of phenylglycine in deuterated solvents ACN:H2O (1:2) and DMSO in the presence of formic acid and sodium hydroxide

A saturated L-phenylglycine (PG) solution (50 mg/mL; 15 µL) in acetonitrile:water (1:2, (v/v)) was derivatised with equimolar amount of 6-aminoquinolyl-N-hydroxysuccinimidyl carbamate (AQC, 3 mg/mL in ACN; 478 µL) for 15 min at 55°C. Purification of PG-AQC was performed via C18 reversed phase chromatography on an Eclipse XDB-C18 (4.6 x 150 mm, 5 µm) column from Agilent using an Agilent 1100 HPLC system with binary pump, autosampler, column oven and faction collector, employing 0.01% formic acid in water as mobile phase A (MPA) and HPLC-grade acetonitrile as mobile phase B (MPB). Injection volume was 50 µL. The collected PG-AQC peak fraction was concentrated on a GeneVac. The purified L-PG-AQC was dissolved in ACN-d₃:D₂O (1:2) and kept as a saturated solution. For the following experiments 100 µL aliquots of this saturated L-PD-AQC solution was used. In case of a change of solvent, 100 µL L-PG-AQC was reduced to dryness in the GeneVac and resuspended in the respective solvent or solution. The following stereoisomerization experiments were performed: a) Purified L-PG-AQC in ACN-d₃:D₂O (1:2) without treatment; samples b) - h) were heated to 95°C for 12 h and differ in their solvent composition: b) L-PG-AQC in ACN-d₃:D₂O (1:2), c) in ACN-d₃:D₂O (1:2) with 0.2 M Na-borated₃ buffer (pH 8.8), d) in ACN-d₃:D₂O (1:2) with 0.5% FA, e) in DMSO-d₆, f) in DMSO-d₆ with 0.2 M Na-borate-d₃ buffer (pH 8.8), g) in DMSO-d₆ with 4x10⁻³% NaOD (w/v) and h) in DMSO-d6 with 4x10⁻⁵% NaOD (w/v). Chiral separation of phenylglycine-AQC (PG-AQC) was performed on a Chiralpak ZWIX (+) column (150 x 4 mm, 3 µm) using an UHPLC-QTOF-ESI(+) system. As mobile phase MeOH:H₂O (98:2; (v/v)) with 9.4 mM NH₄Fa and 9.4 mM FA was used at a flow rate of 300 µL/min and 30°C column temperature. Stereoisomerisation results were visualized via extracted ion chromatograms (XICs) of [PG-AQC+H]⁺: m/z 322.11862 and [PG-AQC-[²H]+H]⁺: m/z 323.12489 as well as the mass spectra for D-PG-AQC at 11.6 min and L-PG-AQC at 12.5 min for the HDX-experiments under investigated stereoisomerisation conditions. In general, in the absence of acid or base (e.g. example a) and e)) no stereoisomerisation was observed. In the presence of acid such as formic acid and base such as sodium hydroxide stereoisomerisation was observed. Concerning solvent type, stereoisomerisation was observed in aqueous hydro-organic solutions as well as organic solvents such as DMSO. The stereoisomerisation results are: b and h < f and g < c and d.

### Example 13: Preparation of stereoisomerisation-tag tagged stereoisomer mixtures

To 180 µL borate buffer (preferably 0.4 M sodium borate buffer with pH 8.0), 10 µL 2.5 mM L-amino acid mixture (0.50 µmol) and 60 µL stereoisomerisation tag (e.g. 3 mg/mL AQC in ACN; 0.63 µmol) were added (final concentration 0.1 mM in 250 µL), and immediately heated to 55°C for 10 min. After centrifugation, ultrasonication for 30 seconds and purging with nitrogen for another 30 seconds, the solution was stereoisomerised for 15 hours at 95°C.

### Example 14: Preparation of stereoisomerisation-tag tagged single stereoisomers

To 160 µL borate buffer (preferably 0.4 M sodium borate buffer with pH 8.0), 20 µL 2.5 mM of a single L-amino acid (50 nmol) and 20 µL stereoisomerisation tag (e.g. 3 mg/mL AQC in ACN; 210 nmol) were added (final concentration 0.25 mM in 200 µL), and immediately heated to 55°C for 10 min. After centrifugation, ultrasonication for 30 seconds and purging with nitrogen for another 30 seconds, the solution was stereoisomerised for 15 hours at 95°C. Purification of AQC-tagged amino acids and removal of the AQC-degradation products 6-aminoquinoline (AMQ) and the bis-aminoquinoline urea was performed via C18 reversed phase chromatography.

### Example 15: Preparation of purified stereoisomer-tagged D- or L-stereoisomers.

The purified AQC-tagged stereoisomer mixture of amino acids were further separated using a ZWIX(+) column (150 x 4 mm, 3 µm). For single stereoisomer separation the short gradient elution method was used, while for simple multi-component mixtures the long gradient elution method was used. For complex multi-component mixtures, a 2D-LC method, combining non-chiral C18-RP-chromatography in the first dimension with stereoisomer separation in the second dimension was used. Alternatively a Chiralpak ZWIX(-), Chiralpak QNAX or a Chiralpak QD-AX column could be used.

### Example 16: Preparation of untagged stereoisomer mixtures

After centrifugation, the stereoisomerised AQC-tagged stereoisomer solution **(Example 13 and Example 14)** was transferred to a microwave tube, purged with nitrogen and irradiated at 300 Watt for 5 min at 95°C to cleave the AQC-tag. Purification of the unlabelled amino acids and removal of the AQC-degradation products 6-aminoquinoline (AMQ) and the bis-aminoquinoline urea was performed via C18 reversed phase chromatography. Note that purification as well as unlabelled amino acid separation can also be performed via hydrophilic interaction liquid chromatography (HILIC).

### Example 17: Preparation of purified untagged D- and/or L-stereoisomers.

The purified label-free stereoisomer mixture of amino acids were further separated using a ZWIX(+) column (150 x 4 mm, 3 µm). For single stereoisomer separation the short gradient elution method was used. For complex multi-component mixtures a 2D-LC method, combining non-chiral HILIC-chromatography in the first dimension with stereoisomer separation in the second dimension was used. Alternatively a Chiralpak ZWIX(-) can be used.

### Example 18: Kit Composition 1a

Kit 1a containing a standard solution with a mixture of *u*-¹²C¹⁴N - proteinogenic L-amino acids, a standard solution of isotopically labelled *u*-¹³C- and *u*-¹³C¹⁵N - proteinogenic L-amino acids, borate buffer (preferably 0.4 M sodium borate buffer with pH 8.0), stereoisomerisation tag and LC-MS grade acetonitrile.

### Example 19: Kit Composition 1b

Kit 1b containing standard solution with *u*-¹²C¹⁴N- proteinogenic DL-amino acids (with preferred stereoisomer ratio range 0.5 - 5%, 5-25% and/or 25-50% D-stereoisomers), a standard solution of stereoisomerisation-tag tagged isotopically labelled *u*-¹³C-or *u*-¹³C¹⁵N- proteinogenic DL-amino acids (with preferred stereoisomer ratio range 0.5 - 5%, 5-25% and/or 25-50% of D-stereoisomers), borate buffer (preferably 0.4 M sodium borate buffer with pH 8.0), stereoisomerisation tag and LC-MS grade acetonitrile.

### Example 20: Kit Composition 1c

Kit 1c containing standard solution with *u*-¹²C¹⁴N- proteinogenic DL-amino acids (with preferred stereoisomer ratio range 0.5 - 5%, 5-25% and/or 25-50% D-stereoisomers), a standard solution of isotopically labelled *u*-¹³C- or *u*-¹³C¹⁵N- proteinogenic DL-amino acids (with preferred stereoisomer ratio range 0.5 - 5%, 5-25% and/or 25-50% of D-stereoisomers) with or without borate buffer (preferably 0.4 M sodium borate buffer with pH 8.0), stereoisomerisation tag and LC-MS grade acetonitrile.

### Example 21: Kit Composition 2

Kit 2 containing a standard solution of one or more stereoisomerisation-tag tagged *u*-¹²C¹⁴N-uncommon L- or D- or DL stereoisomers (of one or more stereoisomers listed in **Claim X** with preferred stereoisomer ratio range 0.5 - 5%, 5-25% and/or 25-50% D-stereoisomers), a standard solution of one or more stereoisomerisation-tag tagged isotopically labelled *u*-¹³C- or *u*-¹³C¹⁵N-uncommon L-, D- or DL-stereoisomers (of one or more stereoisomers listed in Claim X with preferred stereoisomer ratio range 0.5 - 5%, 5-25% and/or 25-50% D-stereoisomers) with or without borate buffer (preferably 0.4 M sodium borate buffer with pH 8.0), stereoisomerisation tag and LC-MS grade acetonitrile.

### Example 22: Kit Composition 3

Kit 3 containing a standard solution of one or more *u*-¹²C¹⁴N-uncommon L- or D- or DL stereoisomers (of one or more stereoisomers listed in **Claim X** with preferred stereoisomer ratio range 0.5 - 5%, 5-25% and/or 25-50% D-stereoisomers), a standard solution of one or more of the respective isotopically labelled *u*-¹³C- or *u*-¹³C¹⁵N-uncommon L-, D- or DL-stereoisomers (of one or more stereoisomers listed in **Claim X** with preferred stereoisomer ratio range 0.5 - 5%, 5-25% and/or 25-50% D-stereoisomers) with or without borate buffer (preferably 0.4 M sodium borate buffer with pH 8.0), stereoisomerisation tag and LC-MS grade acetonitrile.

### Example 23: Kit Composition 4

Kit 4 containing a solution with stereoisomerization-tag tagged isotopically labelled *u*-¹³C- or *u*-¹³C¹⁵N- cell extract (e.g. algal, yeast, mammalian, bacterial) with preferred stereoisomer ratio range 0.5 - 5%, 5-25% and/or 25-50% D-stereoisomers with or without borate buffer (preferably 0.4 M sodium borate buffer with pH 8.0), stereoisomerization tag and LC-MS grade acetonitrile.

### Example 24: Kit Composition 5

Kit 5 containing a solution with isotopically labelled *u*-¹³C- or *u*-¹³C¹⁵N- cell extract (e.g. algal, yeast, mammalian, bacterial) with preferred stereoisomer ratio range 0.5 - 5%, 5-25% and/or 25-50% D-stereoisomers with or without borate buffer (preferably 0.4 M sodium borate buffer with pH 8.0), stereoisomerisation tag and LC-MS grade acetonitrile.

### Conclusion

The amino acid derivatization reagent 6-aminoquinolinyl-N-hydroxysuccinimidyl carbamate is commonly and widely used to introduce a chromophore as well as a highly sensitive fluorophore onto amino functional compounds such as amino acids and peptides or drugs. The here described stereoisomerization (epimerization or racemization) method is inconceivably simple, derivatize your sample with AQC, which takes 10 minutes, then replace the air with inert gas and heat the sample for 30 minutes to up to 15 hours at 95°C on a thermoshaker. Epimerization takes place without loss of glutamine, asparagine or tryptophan. The D-amino acid level depends on the incubation time. It can be stated that if a D-amino acid level between 1-5% is required, an epimerization time of 30 minutes at 95°C is sufficient. Increasing the incubation time to 60 minutes provides a ¹³C¹⁵N-labeled amino acid standard mixture containing 2-10 % D-amino acids, 6 and 15 hours incubation, increases the D-amino acid levels to 15 -30% and 20-45%, respectively. Chiral separation of all proteinogenic amino acids, uncommon amino acids and peptides has been performed on a chiral zwitterionic Chiralpak ZWIX(+) column using an isocratic elution step to separate the critical enantiomer pairs of leucine, isoleucine and proline, the following dual elution gradient provides a fast elution of all other amino acids, with respect to the critical enantiomer pair of glutamic acid. During the gradient run, amino acids are eluted due to an increase in the flow rate from 0.2 mL/min to 0.5 mL/min and due to an increase of the water content. For the separation of peptides the gradient steps have been slightly modified but without change of the mobile phases. The here presented package of an easy and simple procedure to generate tailor-made internal standard with desired D-amino acids composition of natural and uncommon amino acids and D-amino acid level, combined with an universal chiral separation protocol for the zwitterionic Chiralpak ZWIX(+) column, increases flexibility and will in the near future facilitate highly accurate quantitative chiral amino acid metabolomics studies performed in a simple stereoisomerize & shoot approach.

The invention illustratively described herein may suitably be practiced in the absence of any element or elements, limitation or limitations, not specifically disclosed herein. Thus, for example, the terms "comprising", "including," containing", etc. shall be read expansively and without limitation. Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the invention claimed. Thus, it should be understood that although the present invention has been specifically disclosed by exemplary embodiments and optional features, modification and variation of the inventions embodied therein herein disclosed may be resorted to by those skilled in the art, and that such modifications and variations are considered to be within the scope of this invention.

### Materials and Methods

All solvents and formic acid (FA) used in this study were of ultra-LC-MS grade from Carl ROTH (Karlsruhe, Germany). All L-amino acids, DL-amino acids, L-allo-isoleucine, DL-isoleucine, L-allo-threonine, DL-allo-theonine, L-citrulline, trans-4-L-hydroxyproline and 3-aminopyridine were from Sigma Aldrich, Schnelldorf (Germany). Aniline was from Merck (Darmstadt, Germany). A solution of 2.5 mM uniformly labelled *u*-¹³C¹⁵N amino acid containing Metabolomics Standard Mix (MSK-A2-1.2; 2.5 mM in 0.1 M HCl) and a *u*-13C-Algal lyophillized cells (CLM-2065-05) was from Euroisotop GmbH (Saarbrücken, Germany). Note that this ¹³C¹⁵N amino acid standard solution does not contain asparagine, glutamine and tryptophane. The concentration of the in-house prepared ¹²C¹⁴N amino acid standard mix was also 2.5 mM in 0.1 M HCl. Theanine was from TCI (Zwijndrecht, Belgium). Water was purified using a Water Purelab Analytics Purification System from ELGA (Celle, Germany). Nitrogen gas 5.0 quality was from Westfalen Austria GmbH (Muenster, Germany). 6-Aminoquinolinyl-N-hydroxysuccinimidyl carbamate (AQC, AccQ) purchased from Synchem UG & Co. KG (Felsberg/Altenburg, Germany) as well as in-house prepared according to Steven Cohen (EP0533200A1) and De Antonis et.al. (AnalBiochem, 1994, 223, 191). The stereoisomerisation tags, aminophenyl-N-hydroxysuccinimidyl carbamate (AC) and 3-aminopyridyl-N-hydroxysuccinimidyl carbamate (APC) were prepared as described for AQC. Derivatisation reactions with the stereoisomerisation tag were performed on a Thermo-Shaker PHMT Gant-bio for 1.5 mL micro-centrifugation tubes (PSC24N) from Grant Instruments Ltd. (Shepreth, England), while stereoisomerisation reactions were performed with the thermoshaker peqlab thriller for 1.5 mL micro-centrifugation tubes from Peqlab (Erlangen, Germany), but without using the lid. For derivatization, 1.5 mL Crystal Clear microcentrifuge tubes from Starlab (Hamburg, Germany) were used. For stereoisomerisation, Eppendorf® Safe-Lock 1.5 mL microcentrifuge tubes purchased from Sigma-Aldrich Schnelldorf (Germany) were used in combination with parafilm to additionally seal the tubes. Alternatively, for stereoisomerisation, microcentrifuge tubes from Starlab were used in combination with parafilm and Mµlti®-lid locks for microcentrifuge tubes from Carl ROTH (Karlsruhe, Germany).

HPLC-TOF-ESI-QTOF-MS Analysis: Liquid chromatographic separation was performed on an Agilent 1290 UHPLC system with thermostated column oven and an Agilent 1100 system for MS-calibrant delivery from Agilent (Waldbronn, Germany); and a CTC-PAL HTS HTC autosampler from CTC Analytics (Zwingen, Switzerland). All LC-MS measurements were conducted on a TripleTOF 5600+ instrument from Sciex (Ontario, Canada) with Duospray Ion Source operated in electrospray ionisation mode, using sodium acetate clusters for mass calibration. HPLC-QTOF-ESI-MS measurements were performed in positive ionisation mode with a TOF scan combined with information dependent acquisition (IDA) in high sensitivity mode using an inclusion list, which comprised the mass-to-charge ratios of all un-tagged as well as all single and double AQC-tagged amino acid compounds (Supporting Information). The following instrument settings were used: curtain gas (CUR) 40 psi, ion source gas (nebulising gas; GSi) 60 psi, heater gas (drying gas; GS2) 60 psi, ion spray voltage floating (ISVF) 5500 V, source temperature (TEM) 400°C, collision energy (CE) 10 V, declustering potential 100 V. A scan range of *m*/*z* 30 to 2000 with an accumulation time of 250 ms was used for the TOF run. For the IDA experiment with dynamic background subtraction, a CE of 46 V, DP of 100V and an accumulation time of 100 ms were used.

For AQC-, APC- and AC-tagged amino acids and AQC-tagged uncommon amino acids the long hydro-organic dual gradient elution method was used in combination with a Chiralpak ZWIX (+) (150 mm x 4 mm i.d., 3 µm) column using the following chromatographic conditions: mobile phase A (MP A): 9.4 mM NH₄FA, 9.4 mM FA, ACN:MeOH:H₂O (75:25:2; (v/v)); mobile phase B (MP B): MP A : 9.4 mM NH₄FA, 9.4 mM FA in H₂O (1:1, (v/v); isocratic run: MP A 0-35 min at 0.2 mL/min; dual gradient mode: gradient 1 (flow rate): 0-45 min 0.2 mL/min, 45-50 min 0.2-0.5 mL/min and 50-65 min 0.5 mL/min; gradient 2 (hydro-organic): 35¬45 min 0% MP B to 40% MP B, 45-50 min to 60% B, 50-60 min to 100% B and 60-65 min 100%B, followed by column re-equilibration to 100% MP A at 0.2 mL/min. Column temperature was kept at 30°C and sample injection volume were uniformly 2.5 µL.

For AQC-tagged amino acids, non-tagged amino acids and the AQC-tagged RGD-peptide, the short gradient elution method was used in combination with a Chiralpak ZWIX (+) (150 mm x 4 mm i.d., 3 µm) column employing the following chromatographic conditions: mobile phase A (MP A): 9.4 mM NH₄FA, 9.4 mM FA, MeOH:H₂O (98:2; (v/v)); mobile phase B (MP B): MP A : 9.4 mM NH₄FA, 9.4 mM FA in H₂O (1:1, (v/v); 0-9 min 100% MP-A at 700 µL/min, 9-20 min to 100 % MP-B and 20-25 min at 100% MP-B, followed by re-equilibration to 100% MP-A. Injection volume was 2.5 µL, if not otherwise stated.

For enantiomer separation of phenylglycine-AQC (PG-AQC) on a ChiralPAK ZWIX (+) column (150 x 4 mm, 3 µm), the mobile phase MeOH:H₂O (98:2; (v/v)) with 9.4 mM NH₄Fa and 9.4 mM FA was used at a flow rate of 300 µL/min and 30°C column temperature.

## Claims

1. A method for the stereoisomerization (racemization or epimerization) of a chiral compound, the method comprising the steps:
a) providing a mixture comprising:
(aa) the chiral compound which contains at least one stereogenic carbon atom;
(bb) a stereoisomerization tag; and
(cc) optionally a buffer;
wherein the molar concentration of the stereoisomerization tag in the mixture is greater than the molar concentration of the chiral compound; and
(b.1) heating the mixture to a temperature from 50 to 200°C for 30 minutes to 24 hours; or
(b.2) treating the mixture in a microwave at 200 to 400 W for 1 min to 6 h, at a temperature from 50 to 300°C.

2. The method according to claim 1, wherein the heating in step (b.1) is at a temperature range from 55 to 95°C.

3. The method according to claim 1 or 2, wherein the chiral compound has the following formula (I): 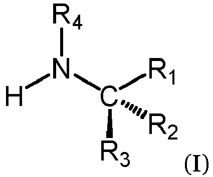 wherein the groups R₁, R₂ and R₃ are chemically not identical, may form a ring, and are independently selected from the group consisting of hydrogen, linear or branched alkyl group, aromatic groups, heteroaromatic groups, carboxyl group, phosphonic acid group, sulfonic acid group, ester group, amide group, carbamate group or urea group,
wherein R₄, is selected from the group consisting of hydrogen, linear or branched alkyl group, aromatic groups, hetero-aromatic groups, carboxyl group, phosphonic acid group, sulfonic acid group, ester group, amide group, carbamate group or urea group;
preferably, wherein the chiral compound is selected from the group consisting of amino acids, preferably proteinogenic amino acids, but includes non-proteinogenic, non-ribosomal and synthetic amino acids.

4. The method according to claim 3, comprising a step of activating the chiral compound to achieve an intermediate compound of the following formula (IIa): 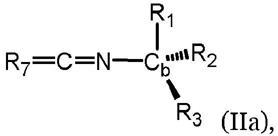 wherein R₇ may be selected from N, O, S or Se; or preferably is any of the following compounds (II) and (III): 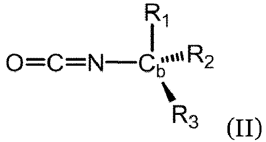 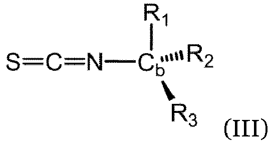 wherein the stereoisomerization-tag comprises at least one of the following compounds 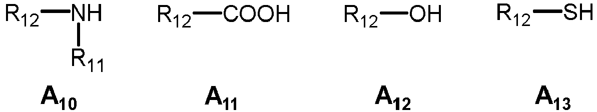 wherein R₁₁ and R₁₂ are independently selected from the group consisting of hydrogen, linear or branched alkyl groups, aromatic groups, heteroaromatic groups, carboxyl group, phosphonic acid group, sulfonic acid group, ester group, amide group carbamate group or urea group.

5. Method according to any one of claims 1 to 3, wherein the stereoisomerization tag results from or comprises or consists of at least one compound selected from the group consisting of compounds A₁ to A₉: 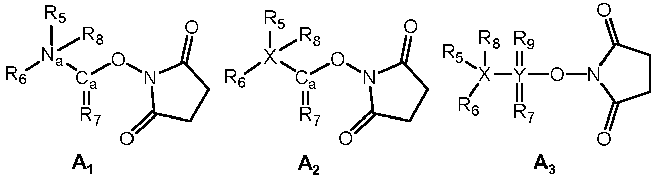 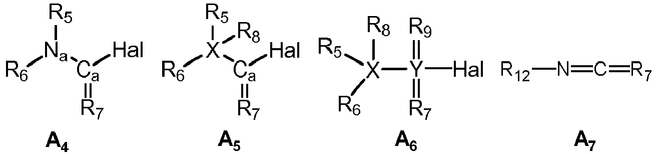 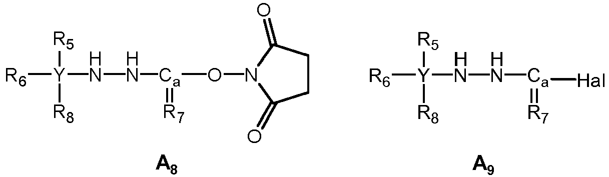
wherein R₄, R₅, R₆, R₈ and R₁₂ are independently selected from the group consisting of hydrogen, linear or branched alkyl groups, aromatic groups, heteroaromatic groups, carboxyl group, phosphonic acid group, sulfonic acid group, ester group, amide group, carbamate group or urea group;
and
Hal and OSu are leaving groups
R₇ and R₉ may be independently selected from N, O, S or Se;
X may be N, O, S, Se, or C; and
Y may be C, S, P, or Se.

6. The method according to claim 5, wherein the stereoisomerization-tag is selected from aminophenyl-N-hydroxysuccinimidyl carbamate (AC), 3-aminopyridyl-N-hydroxysuccinimidyl carbamate (APC) and, preferably, 6-aminoquinolinyl-N-hydroxysuccinimidyl carbamate (AQC, AccQ).

7. The method according to any one of the preceding claims, further comprising a step of removing the stereoisomerization-tag from the stereoisomerized compound.

8. The method according to any one of the preceding claims, wherein (i) the chiral compound and/or (ii) the stereoisomerization-tag, is isotopically labelled with a stable, meta-stable or unstable isotope, the isotope being preferably selected from ¹¹C, 12C, ¹³C, ¹⁴C, ¹⁴N, ¹⁵N, ¹⁶O, ¹⁷O, ¹⁸O, ³²S, ³³S, ³⁴S, ³⁵S, ³⁶S, ⁷⁴Se, ⁷⁶Se, ⁷⁷Se, ⁷⁸Se, ⁷⁹Se, ⁸⁰Se, ⁸²Se, ³¹P, ³²P, ³³P, ¹²³I, ¹²⁷I, ¹²⁹I, ¹³¹I, ¹³⁵I, ¹⁸F, ¹⁹F, ¹H, ²H and ³H, most preferably using the isotopes ¹²C, ¹⁴N, ¹⁶O, ³²S, ³¹P, ¹²⁷I, ¹⁹F and ¹H.

9. A method for producing a
(i) (R) stereoisomer with respect to the stereogenic center C of a chiral compound having one or more stereogenic centers according to formula I in claim 3, the method comprising performing the method steps of any one of claims 1 to 8; wherein the stereogenic center C of the chiral compound of formula I is in (S) configuration; or
(ii) (S) stereoisomer with respect to the stereogenic center C of a chiral compound having one or more stereogenic centers according to formula I in claim 3, the method comprising performing the method steps of any one of claims 1 to 8; wherein the stereogenic center C of the chiral compound of formula (I) is in (R) configuration.

10. A method of producing a predefined mixture of stereoisomers (or epimers), of a chiral compound according to formula (I) in claim 3, wherein the predefined mixture of stereoisomers comprises a predetermined ratio Y of (R)/(S)-stereoisomers of the stereogenic center C of the chiral compound according to formula (I);
wherein the predefined mixture of stereoisomers is obtained by performing the steps of a method according to any one of claims 1 to 8;
wherein the mixture of step a) in the method according to any one of claims 1 to 8 comprises the chiral compound in a ratio X of (R)/(S) enantiomers of the stereogenic center C, wherein X < Y; and
wherein the temperature and/or time in step (b.1) or the power (W) and time in step (b.2), is selected to epimerize a sufficient amount of (R)-stereoisomer to the (S)-stereoisomer to arrive at the predetermined ratio Y of (R)/(S)-stereoisomers in the reaction mixture and to thereby obtain the predefined mixture of stereoisomers.

11. A method of producing a predefined mixture of stereoisomers (or epimers), of a chiral compound according to formula (I) in claim 3, wherein the predefined mixture of stereoisomers comprises a predetermined ratio Y of (R)/(S)-stereoisomers of the stereogenic C of the chiral compound according to formula (I);
wherein the predefined mixture of stereoisomers is obtained by performing the steps of a method according to any one of claims 1 to 8;
wherein the mixture of step a) in the method according to any one of claims 1 to 8 comprises the chiral compound in a ratio X of (R)/(S) enantiomers of the stereogenic C, wherein X > Y; and
wherein the temperature and/or time in step (b.1) or the power (W) and time in step (b.2), is selected to epimerize a sufficient amount of (S)-stereoisomer to the (R)-stereoisomer to arrive at the predetermined ratio Y of (R)/(S)-stereoisomers in the reaction mixture and to thereby obtain the predefined mixture of stereoisomers.

12. The method according to claim 10 or 11, wherein the predefined mixture of stereoisomers (or epimers) is prepared within a biological sample, which is preferentially isotopically labeled, and wherein the predefined mixture of stereoisomers is used as an external or internal standard for detecting, identifying and/or quantifying one or more chiral biological compounds in a biological sample.

13. The method according to claim 10 or 11, wherein the predefined mixture of stereoisomers (or epimers) is prepared in the presence of oxygen.

14. A predefined mixture of stereoisomers (or epimers) of a chiral compound, wherein the mixture of stereoisomers (or epimers) is obtainable by a method according to any one of claims 10 to 13.

15. A use of a predefined mixture of stereoisomers according to claim 14 in a method of detecting, identifying and/or quantifying one or more chiral biological compounds in a biological sample, for example as an internal standard in the method of detecting and/or quantifying said chiral biological compounds.

16. A method of detecting, identifying and/or quantifying (R)-and/or (S)-stereoisomers of a chiral compound (analyte) in a biological sample from a subject, comprising:
(a) preparing a sample by adding a predefined mixture of preferentially isotopically labeled stereoisomers according to any one of claims 10 to 13 as internal standard into the biological sample;
(b) analyzing the eluent for the presence of said preferentially isotopically labelled enantiomers as internal standard together with analyte enantiomers that are to be identified and/or quantified, for example using a mass analyzer.

17. A compound, which is a stereoisomer, selected from:
(i) a proteinogenic amino acids selected from D-¹³C₅,¹⁵N-Valine, D-¹³C₆-Leucine, D-¹³C₆,¹⁵N-Leucine, D-¹³C₆-Isoleucine, D-¹³C₆,¹⁵N-Isoleucine, DL-¹³C₆-Isoleucine, DL-¹³C₆,¹⁵N-Isoleucine, D-¹³C₅-Methionine, D-¹³C₅,¹⁵N-Methionine, DL-¹³C₅-Methionine, DL-¹³C₅,¹⁵N-Methionine, D-¹³C₅-Proline, D-¹³C₅,¹⁵N-Proline, DL-¹³C₅-Proline, D-¹³C₃-Serine, D-¹³C3,¹⁵N-Serine, D-¹³C₄-Threonine, D-¹³C₄,¹⁵N-Threonine, DL-¹³C₄-Threonine, DL-¹³C₄,¹⁵N-Threonine, D-¹³C₃-Cystein, D-¹³C₃,1⁵N-Cystein, D-¹³C6-Cystine, D-¹³C6,¹⁵N-Cystine, D-¹³C₉-Phenylalanine, DL-¹³C₉-Tyrosine, DL-¹³C₉,1⁵N-Tyrosine, D-¹³C₁₁-Tryptophane, D-¹³C₁₁,¹⁵N₂-Tryptophane, DL-¹³C₁₁-Tryptophane, DL-¹³C₁₁, ¹⁵N₂-Tryptophane, DL-¹³C₅,¹⁵N-Glutamic acid, D-¹³C₄,¹⁵N-Aspartic acid, DL-¹³C₄,¹⁵N-Aspartic acid, D-¹³C₅-Glutamine, DL-¹³C₅-Glutamine, DL-¹³C₅,¹⁵N-Glutamine, DL-¹³C₅,¹⁵N₂-Glutamine, D-¹³C₄,¹⁵N₂-Asparagine, DL-¹³C₄-Asparagine, DL-¹³C₄,¹⁵N-Asparagine, DL-¹³C₄,¹⁵N2-Asparagine, D-¹³C₆-Histidine, D-¹³C₆,¹⁵N-Histidine, D-¹³C₆,¹⁵N₂-Histidine, D-¹³C₆,¹⁵N₃-Histidine, DL-¹³C₆-Histidine, DL-¹³C₆,¹⁵N-Histidine, DL-¹³C₆,¹⁵N₂-Histidine, DL-¹³C₆,¹⁵N₃-Histidine, D-¹³C₆,¹⁵N-Lysine, D-¹³C₆,¹⁵N₂-Lysine, DL-¹³C₆,¹⁵N-Lysine, DL-¹³C₆,¹⁵N₂-Lysine, D - ¹³C₆-Arginine, D-¹³C₆,¹⁵N-Arginine, D-¹³C₆,¹⁵N₂-Arginine, D-¹³C₆,¹⁵N₃-Arginine, D-¹³C₆,¹⁵N₄-Arginine, DL-¹³C₆-Arginine, DL-¹³C₆,¹⁵N-Arginine, DL-¹³C₆,¹⁵N₂-Arginine, DL-¹³C₆,¹⁵N₃-Arginine;
(ii) an oxidation product selected from Met: L-¹³C₅,¹⁵N-Methionine sulfoxide (S-*(S)),* L-¹³C₅,¹⁵N-Methionine sulfoxide (*S*-(*R*)), D-¹³C₅,¹⁵N-Methionine sulfoxide (R-(*S*)), D-¹³C₅,¹⁵N-Methionine sulfoxide (R-(*R*)), DL-¹³C₅,¹⁵N-Methionine sulfoxide (RS-(*S*)), DL-¹³C₅,¹⁵N-Methionine sulfoxide (RS-(*R*)), L-¹³C₅,¹⁵N,¹⁸O-Methionine sulfoxide (S-(*S*)), L-¹³C₅,¹⁵N,¹⁸O-Methionine sulfoxide (S-(*R*)), D-¹³C₅,¹⁵N,¹⁸O-Methionine sulfoxide (R-(*S*)), D-¹³C₅,¹⁵N,¹⁸O-Methionine sulfoxide (R-(*R*)), DL-¹³C₅,¹⁵N,¹⁸O-Methionine sulfoxide (RS-(*S*)), DL-¹³C₅,¹⁵N,¹⁸O-Methionine sulfoxide (RS-(*R*)), L-¹³C₅,¹⁵N-Methionine sulfone (S), D-¹³C₅,¹⁵N-Methionine sulfone (R), DL-¹³C₅,¹⁵N-Methionine sulfone (RS), L-¹³C₅,¹⁵N,¹⁸O-Methionine sulfone (S), D-¹³C₅,¹⁵N,¹⁸O-Methionine sulfone (R), DL-¹³C₅,¹⁵N,¹⁸O-Methionine sulfone (RS), L-¹³C₅,¹⁵N,¹⁸O₂-Methionine sulfone (S), D-¹³C₅,¹⁵N,¹⁸O₂-Methionine sulfone (R), DL-¹³C₅,¹⁵N,¹⁸O₂-Methionine sulfone (RS), L-¹³C₅-Methionine sulfoxide (S-(*S*)), L-¹³C₅-Methionine sulfoxide (S-(*R*)), D-¹³C₅-Methionine sulfoxide (R-(*S*)), D-¹³C₅-Methionine sulfoxide (R-(*R*)), DL-¹³C₅-Methionine sulfoxide (RS-(*S*)), DL-¹³C₅-Methionine sulfoxide (RS-(*R*)), L-¹³C₅,¹⁸O-Methionine sulfoxide (S-(*S*)), L-¹³C₅,¹⁸O-Methionine sulfoxide (S-(*R*)), D-¹³C₅,¹⁸O-Methionine sulfoxide (R-(*S*)), D-¹³C₅,¹⁸O-Methionine sulfoxide (R-(*R*)), DL-¹³C₅,¹⁸O-Methionine sulfoxide (RS-(*S*)), DL-¹³C₅,¹⁸O-Methionine sulfoxide (RS-(*R*)), L-¹³C₅-Methionine sulfone (S), D-¹³C₅-Methionine sulfone (R), DL-¹³C₅-Methionine sulfone (RS), L-¹³C₅,¹⁸O-Methionine sulfone (S), D-¹³C₅,¹⁸O-Methionine sulfone (R), DL-¹³C₅,¹⁸O-Methionine sulfone (RS), L-¹³C₅,¹⁸O₂-Methionine sulfone (S), D-¹³C₅,¹⁸O₂-Methionine sulfone (R), DL-¹³C₅,¹⁸O₂-Methionine sulfone (RS);
(iii) a decomposition product selected from Trp: L-¹³C₁₀,¹⁵N₂-Kynurenine, D-¹³C₁₀,¹⁵N₂-Kynurenine, DL-¹³C₁₀,¹⁵N₂-Kynurenine, L-¹³C,O,¹⁵N-Kynurenine, D-¹³C₁₀,¹⁵N-Kynurenine, DL-¹³C₁₀,¹⁵N-Kynurenine, L-¹³C₁₀-Kynurenine, D-¹³C₁₀-Kynurenine, DL-¹³C₁₀-Kynurenine;
(iv) an uncommon amino acid selected from D-¹³C₆-allo-Isoleucine, D-¹³C₆,¹⁵N-allo-Isoleucine, DL-¹³C₆-allo-Isoleucine, DL-¹³C₆,¹⁵N-allo-Isoleucine, D-¹³C₄-allo-Threonine, D-¹³C₄¹⁵N-allo-Threonine, DL-¹³C₄-allo-Threonine, DL-¹³C₄¹⁵N-allo-Threonine, D-¹³C₄-Homoserine, D-¹³C₄¹⁵N-Homoserine, DL-¹³C₄-Homoserine, DL-¹³C₄¹⁵N-Homoserine, D-¹³C₄,¹⁵N-Homocystein, D-¹³C₄-Homocystein, DL-¹³C₄,¹⁵N-Homocystein, DL-¹³C₄-Homocystein, D-¹³C₈¹⁵N₂-Homocystine, D-¹³C₈¹⁵N-Homocystine, D-¹³C₈-Homocystine, DL-¹³C₈N₂-Homocystine, DL-¹³C₈N-Homocystine, DL-¹³C₈-Homocystine, D-¹³C₅,¹⁵N₂-Ornithine, D-¹³C₅,¹⁵N-Ornithine, D-¹³C₅-Ornithine, DL-¹³C₅,¹⁵N₂-Ornithine, DL-¹³C₅,¹⁵N-Ornithine, DL-¹³C₅-Ornithine, D-¹³C₆,¹⁵N₃-Citrulline, D-¹³C₆,¹⁵N₂-Citrulline, D-¹³C₆,¹⁵N-Citrulline, D-¹³C₆-Citrulline, DL-¹³C₆,¹⁵N₃-Citrulline, DL-¹³C₆,¹⁵N₂-Citrulline, DL-¹³C₆,¹⁵N-Citrulline, DL-¹³C₆-Citrulline, D-¹³C₅,¹⁵N-Norvaline, D-¹³C₅-Norvaline, DL-¹³C₅,¹⁵N-Norvaline, DL-¹³C₅-Norvaline, D-¹³C₆,¹⁵N-Norleucine, D-¹³C₆-Norleucine, DL-¹³C₆,¹⁵N-Norleucine, DL-¹³C₆-Norleucine, D-¹³C₄,¹⁵N-2-Aminobutyric acid, D-¹³C₄-2-Aminobutyric acid, DL-¹³C₄,¹⁵N-2-Aminobutyric acid, DL-¹³C₄-2-Aminobutyric acid, D-¹³C₄,¹⁵N-Aminoisobutyric acid, D-¹³C₄-Aminoisobutyric acid, DL-¹³C₄,¹⁵N-Aminoisobutyric acid, DL-¹³C₄-Aminoisobutyric acid, D-¹³C₁₀,¹⁵N₃-Hypusine *(R)*, D-¹³C₁₀,¹⁵N₃-Hypusine *(S)*, D-¹³C₁₀,¹⁵N₂-Hypusine *(R)*, D-¹³C₁₀,¹⁵N₂-Hypusine (*S*), D-¹³C₁₀,¹⁵N-Hypusine (*R*), D-¹³C₁₀,¹⁵N-Hypusine *(S)*, DL-¹³C₁₀,¹⁵N₃-Hypusine (R), DL-¹³C₁₀,¹⁵N₃-Hypusine (S), DL-¹³C₁₀,¹⁵N₂-Hypusine (R), DL-¹³C₁₀,¹⁵N₂-Hypusine (S), DL-¹³C₁₀,¹⁵N-Hypusine (R), DL-¹³C₁₀,¹⁵N-Hypusine (S), D-¹³C₁₀,¹⁵N₃-Deoxyhypusine, D-¹³C₁₀,⁵N₂-Deoxyhypusine, D-¹³C₁₀,¹⁵N-Deoxyhypusine, D-¹³C₁₀-Deoxyhypusine, DL-¹³C₁₀,¹⁵N₃-Deoxyhypusine, DL- ¹³C₁₀,¹⁵N₂-Deoxyhypusine, DL-¹³C₁₀,¹⁵N-Deoxyhypusine, DL-¹³C₁₀-Deoxyhypusine.

18. A racemic or non-racemic composition comprising a stereoisomer according to claim 17, with or without racemization tag, preferably, wherein the racemic or non-racemic composition is in the form of a pharmaceutical composition.
